# FASCICULE DE BREVET EUROPEEN

(11) **EP 0 332 488 B1**
(45) Date de publication et mention de la délivrance du brevet: **27.11.1996**
(21) Numéro de dépôt: 89400468.8
(22) Date de dépôt: 20.02.1989
(51) Int. Cl.: C12N 15/00, C12N 1/20, C12P 13/08, C12P 21/02

(54) **Procédé pour l'intégration d'un gène choisi sur le chromosome d'une bactérie et bactérie obtenue par ledit procédé**
Verfahren zur Integration eines bestimmten Gens ins bakterielle Chromosom und durch dieses Verfahren erhaltenes Bakterium
Method for the integration of a chosen gene into a bacterial chromosome, and bacterium obtained by said method

(30) Priorité: 22.02.1988 FR 8802078
(43) Date de publication de la demande: 13.09.1989
(73) Titulaire: EUROLYSINE, F-75009 Paris (FR)
(72) Inventeur: Richaud, François,, F-75013 Paris (FR); Jarry, Bruno,, F-75017 Paris (FR); Takinami, Koichi,, F-92290 Chatenay Malabry (FR); Kurahashi, Osamu,, F-75016 Paris (FR); Beyou, Anne,, F-75013 Paris (FR)
(74) Mandataire: Warcoin, Jacques

(56) Documents cités:
- EP-A- 0 019 877
- EP-A- 0 091 723
- EP-A- 0 127 328
- EP-A- 0 166 628
- EP-A- 0 166 659
- EP-A- 0 200 708
- WO-A-88/01646
- US-A- 4 670 388
- GENE, vol. 42, 1986, pages 185-192, Elsevier Science Publishers B.V.(Biomedical Division); P. RATET et al.:
- SCIENCE, vol. 239, 15 janvier 1988, pages 280-281; J.D. BOEKE et al.
- AGRICULTURAL AND BIOLOGICAL CHEMISTRY, vol. 48, no. 9, septembre 1984, pages 2233-2237, Tokyo, JP; K. MIWA et al.:
- BIOTECHNOLOGY, vol. 4, no. 5, mai 1986, pages 446-449, New York, US; G.F. BARRY: "Permanent insertion of foreign genes into the chromosomes of soil bacteria"
- THE EMBO JOURNAL, vol. 4, no. 4, 1985, pages 891-898, IRL Press Ltd, Oxford, GB; S.E. STACHEL et al.
- CHEMICAL ABSTRACTS, vol. 103, no. 19, novembre 1985, page 218, rèsumé no. 155267m, Columbus, Ohio, US; & JP-A-60 118 188 (NODA INSTITUTE FOR SCIENTIFIC RESEARCH) 25-06-1985
- Lichtenstein and Brenner; Nature, vol.297 (1982) pp.601-603.
- Grinter; Gene, vol.21 (1983) pp.133-143.

## Description

La présente invention concerne une nouvelle méthode pour l'amplification et la stabilisation du nombre de copies de gènes choisis dans un microorganisme.

L'invention est illustrée au moyen d'exemples cités plus loin dans lesquels la séquence codant pour une enzyme, la béta-galactosidase, et les gènes codant pour trois enzymes intervenant dans la biosynthèse de la L-thréonine sont transférés à l'intérieur du chromosome d'un microorganisme.

Ces gènes se sont avérés être fixés de façon stable dans le chromosome. Les souches résultantes sont alors de meilleures productrices, des enzymes correspondantes pour le premier cas ou d'acides aminés L-thréonine pour le second cas, que les souches parentales.

Les bactéries sont capables de synthétiser des principes actifs utiles tels que des enzymes et des acides aminés. La production par fermentation fait intervenir des processus biochimiques complexes contrôlés par de multiples gènes et produits de gènes. Les produits de la fermentation sont fabriqués en quantités relativement faibles. Pour améliorer cette production par fermentation, il serait utile d'augmenter le nombre de copies des gènes intervenant, d'une manière telle que le nombre de ces copies puisse être contrôlé et stabilisé dans le chromosome ou les épisomes du microorganisme.

Plusieurs méthodes ont été décrites dans la littérature scientifique selon lesquelles le nombre de copies d'un gène peut être amplifié dans un microorganisme, en particulier au moyen de vecteurs circulaires plasmidiques. Néanmoins, le nombre de copies obtenues par ces méthodes est hautement variable et dépend du vecteur particulier, de la longueur du gène choisi et des conditions physiologiques particulières du microorganisme. Aucune de ces méthodes ne concerne l'amplification du gène jusqu'à un niveau choisi, en évitant un excès non indispensable de copies, et ceci avec une fixation stable de ces copies dans le chromosome.

Divers documents suggèrent l'utilisation de transposons à titre de vecteurs dans les bactéries ou d'autres organismes, notamment :
- le brevet US-A-4 670 388,
- le brevet EP-A-0 200 708,
- le brevet EP-A-0 091 723,
- GENE, Vol. 42 (1986), pages 185-192,
- THE EMBO JOURNAL, Vol. 4, n° 4, 1985, pages 891-898,
- BIOTECHNOLOGY, Vol. 4, n° 5, mai 1986, pages 446-449, New York, US,

Aucun de ces documents ne décrit toutefois un procédé permettant d'obtenir une intégration stable d'une séquence d'ADN déterminée avec un nombre de copies défini.

Un but de l'invention est de décrire une méthode pour amplifier et fixer de manière stable les copies de gènes choisis dans un microorganisme avec plus de flexibilité et plus de sécurité que ce qui était possible jusqu'à présent. Ce but ainsi que d'autres objectifs de l'invention tels qu'envisagés plus loin ont été réalisés en fournissant, par exemple, à la bactérie Escherichia coli un élément transposable à large spectre d'hôtes dérivé du phage Mu et dans lequel les gènes de la transposase ne peuvent s'exprimer ou sont délétés. Un tel transposon est un transposon désarmé. Dans des conditions génétiques réversibles particulières, faciles à reproduire pour des organismes du même genre ou d'un genre différent, ce transposon et les gènes, qui peuvent y être introduits par les technologies de génie génétique, peuvent être conçus pour se multiplier au sein du chromosome du microorganisme. Quand les conditions génétiques particulières sont supprimées, les différentes copies du transposon restent fixées de manière stable à l'intérieur du chromosome.

La présente invention concerne un procédé pour l'intégration d'un gène choisi ou d'une séquence d'ADN déterminée (à l'intérieur d'une bactérie) dans une séquence d'ADN telle qu'un chromosome ou un épisome dans lequel :
a) ledit gène choisi, ou la séquence d'ADN choisie, est cloné à l'intérieur d'un transposon désarmé, à l'extérieur des parties essentielles du transposon,
b) ledit transposon est intégré dans la séquence d'ADN à l'intérieur de la bactérie.

Plus particulièrement, le procédé selon l'invention est utilisé pour amplifier le nombre de copies de gènes choisi afin d'obtenir un nombre déterminé de copies. La présente invention a donc plus précisément pour objet un procédé pour l'intégration d'un nombre déterminé de copies d'un gène choisi ou d'une séquence d'ADN déterminée dans une séquence d'ADN telle que le chromosome ou l'épisome d'une bactérie, caractérisé en ce que :
a) ledit gène choisi ou la séquence d'ADN choisie est cloné à l'intérieur d'un transposon à l'extérieur des parties essentielles du transposon, ledit transposon étant désarmé,
b) ledit transposon est intégré dans la séquence d'ADN telle que le chromosome ou l'épisome de ladite bactérie,
c) ledit transposon désarmé est complémenté de manière à pouvoir transposer plusieurs fois et la complémentation est ensuite arrêtée après un nombre déterminé de transpositions.

Selon l'invention, on peut utiliser tout transposon qui transpose à l'intérieur d'un site quelconque dans l'ADN bactérien.

On entend désigner dans le présent texte par "transposons" aussi bien les "transposons vrais" que les phages ayant la capacité de transposer.

Des transposons préférés sont Tn3, Tn5, Tn7, Tn10, Tn903, TnHoHo, IS 1, IS 10, IS 50 et les phages MudI et MudII. On préfère tout particulièrement les phages MudI et MudII.

L'utilisation de transposons désarmés qui sont des transposons incapables de transposer par eux-mêmes permettent d'obtenir une intégration stable. Pour un tel usage, les transposons utilisés n'ont pas de gènes pour la transposase ou ces gènes ne sont pas actifs dans ladite bactérie. On préfère tout particulièrement un transposon dépourvu des gènes codant pour les transposases, mais il est aussi possible d'utiliser un transposon dans lequel les transposases, agissant en trans sont réprimées ou inactives dans des conditions normales.

Selon l'invention on peut utiliser n'importe quelle bactérie dans laquelle les transposons décrits ci-dessus peuvent transposer.

Les bactéries préférées sont des membres de la famille des Entérobactériacées, par exemple Escherichia coli. Citrobacter freundii, Erwinia herbicola ou E. chrysanthemi, Salmonella thyphimurium, Shigella sonneii, Enterobacter cloacae, Serratia marcescens ou des membres de la famille des Rhizobiacées tels que Agrobacterium tumefaciens ou des membres de la famille Pseudomonas tels que Pseudomonas putida par exemple.

Par gène choisi, on définit un gène qui n'est pas normalement présent dans ledit transposon ou qui n'est pas présent dans la bactérie hôte.

Comme gène choisi, on peut utiliser un gène cloné ou un gène hybride, un segment de gène ou un gène fabriqué synthèse. Le gène peut s'exprimer dans la bactérie hôte et être d'origine animale, végétale ou microbienne. Plusieurs gènes peuvent être insérés dans le transposon.

Le gène choisi peut être sous contrôle d'un promoteur qui est, lui aussi, à l'intérieur du transposon et qui assure l'expression dudit gène dans ladite bactérie.

Les gènes clonés sont insérés par des techniques connues de génie génétique ou selon le procédé décrit ci-dessous.

Pour plus de facilité, il est plus simple de partir d'un plasmide contenant le transposon désarmé. Le transposon est désarmé par délétion ou mutation.

Le plasmide contenant le transposon est isolé de la bactérie et clivé avec des enzymes de restriction appropriées. Les gènes choisis sont aussi extraits de leur hôte puis purifiés après clivage au moyen d'enzymes de restriction. Le clonage d'un gène marqueur tel qu'un gène de résistance à un antibiotique (npt par exemple) ou un autre "reporter" (lacZ par exemple) en même temps que les gènes insérés est préférable pour la sélection des évènements de transposition. On évalue le nombre de transpositions par la détermination de l'expression du gène marqueur. Ladite expression du gène marqueur peut être déterminée par la résistance à un antibiotique quand le gène marqueur est un gène de résistance à un antibiotique; l'expression du gène marqueur peut aussi être déterminée par la coloration d'un substituant quand le gène marqueur code pour une activité enzymatique qui peut être évaluée par un essai colorimétrique. Les différents fragments sont recombinés par ligation in vitro et transformés dans la bactérie réceptrice appropriée. Les transformants sont sélectionnés et ensuite analysés pour déterminer que la construction est convenable. Le transposon désarmé composite résultant peut être placé dans d'autres souches par transformation ou transduction. Des méthodes conventionnelles de génie génétique sont décrites dans "Molecular cloning, a laboratory manual" (Maniatis et al., Cold Spring Harbor Laboratory, New York, 1986).

Lorsque le procédé de l'invention est utilisé pour amplifier le nombre de copies du gène choisi, l'intégration d'un nombre déterminé de copies dudit gène choisi alors, au cours du procédé décrit ci-dessus utilisant un transposon désarmé et après l'intégration, ledit transposon incapable de transposer est complémenté de manière à pouvoir transposer plusieurs fois et la complémentation est ensuite arrêtée après un nombre déterminé de transpositions.

Ladite complémentation peut être réalisée de différentes manières. Si le transposon est réellement désarmé, c'est-à-dire s'il ne possède pas de transposase, cette dernière peut être introduite dans la cellule hôte grâce à un plasmide ou un phage codant pour ladite transposase.

Si l'expression des transposases est réprimée, il est possible d'inactiver le répresseur, dans le cas d'un répresseur sensible à la chaleur l'élévation de la température aura pour résultat l'expression des transposases.

Une fois inséré dans l'ADN receveur, le transposon fabriqué peut être amplifié par transposition sur le génome au moyen de l'inactivation du répresseur conduisant à l'expression des gènes de la transposase.

Le transposon dans lequel les gènes de la transposase manquent peut être amplifié par introduction d'un plasmide ou d'un phage contenant des gènes de transposase agissant en trans.

Lorsque la complémentation est effectuée par introduction d'un élément extra chromosomique tel qu'un plasmide ou phage notamment, cette complémentation peut être effectuée simultanément à l'étape d'intégration.

Les transposons amplifiés restent de manière stable dans l'ADN bactérien dans les conditions de culture habituelles. On préfère utiliser un transposon désarmé pour stabiliser les gènes choisis. N'importe quel ADN dans une cellule bactérienne peut être la cible de la transposition. On préfère tout particulièrement les ADN qui restent de manière stable dans la cellule bactérienne telle que le chromosome ou un plasmide stable.

Selon une réalisation préférée de l'invention, le transposon désarmé utilisé est MudII1734 (Castilho et al., J. Bact., 158, 488, 1984) qui est présent comme prophage défectif dans une souche d'E. coli(POII1734) déjà lysogène pour un phage mutant Mucts. Cette souche cultivée à basse température est brièvement chauffée et le lysat résultant est utilisé pour la transduction avec une souche réceptrice convenable. Les bactéries résistantes à l'antibiotique adéquat et qui ne produisent pas de phage Mucts ne contiennent que le transposon dans leur chromosome.

Le MudII1734 est inséré sur un plasmide, puis l'intérieur du Mud peut être modifiée (selon la partie de la description ci-dessus page 3 ligne 27 à page 4 ligne 9).

Le plasmide contenant le Mud modifié est transformé dans une souche lysogénique pour Mucts. Le lysat de phage est obtenu après élévation de la température. Ce lysat de phage est utilisé pour transduire le Mud modifié sur le chromosome d'une bactérie déterminée.

Pour amplifier le Mud modifié, la souche qui porte le transposon défectif sur son chromosome est transformée avec un plasmide qui contient les deux gènes de transposase, à savoir les gènes A et B, ou est infectée par le phage Mu.

Dans le cas d'amplification par un plasmide, le plasmide contenant les gènes A et B est maintenu dans la bactérie par l'utilisation d'une sélection (résistance à un antibiotique). Les bactéries qui poussent en présence de cet antibiotique et en même temps synthétisent en grande quantité le produit d'un gène situé dans le transposon sont sélectionnées. Les bactéries qui expriment ces deux phénotypes peuvent contenir plusieurs copies supplémentaires du transposon sur leur chromosome.

Les bactéries qui ont perdu le plasmide ou qui ne sont pas lysogéniques pour le phage peuvent être obtenues naturellement.

La détermination du nombre de copies de transposon peut être effectuée par sélection des bactéries décrites ci-dessus selon le niveau d'expression d'un gène marqueur dans le transposon.

Le nombre de copies du transposon contenant un gène de résistance à un antibiotique peut être estimé selon la résistance de la bactérie à l'antibiotique.

Le nombre de copies du transposon incluant l'operon lac peut être estimé selon l'intensité de la coloration de colonies bactériennes après dégradation d'un substrat tel que le X-gal (5-bromo-4-chloro-3-indolyl-béta-D-galactoside).

Le nombre de copies du transposon peut être déterminé en utilisant le "Southern blotting" et les techniques d'hybridation ADN-ADN avec des sondes appropriées.

Les gènes choisis amplifiés à l'aide du transposon restent de manière stable dans la mesure où les gènes de transposase ne sont pas présents ou sont inactifs dans la souche bactérienne.

On préfère aussi, dans ce but, introduire des mutations de l'ADN polymérase I ou de l'exonucléase 5′ 3′ ou une mutation déficiente en HU dans la bactérie hôte après l'amplification.

Un aspect important de cette invention est que les souches obtenues sont très stables. Le transposon désarmé ne peut ni transposer davantage ni être délété par aucun mécanisme efficace. Après une culture pendant de nombreuses générations sans pression sélective, les transposons désarmés occupent toujours la même position dans le chromosome.

De plus aucun transfert du transposon désarmé n'est détectable au cours des expériences de coculture.

La présente invention concerne aussi ladite bactérie qui peut être obtenue par le procédé selon l'invention et concerne également un procédé pour la préparation du produit du gène hétérologue comprenant la culture de la bactérie selon l'invention dans un milieu de culture convenable et la récupération dudit produit.

Bien que le gène choisi puisse coder pour un peptide ou une protéine ayant une utilité thérapeutique ou industrielle, une des mises en oeuvre du procédé concerne la préparation de métabolites grâce à l'utilisation de la bactérie obtenue selon le procédé décrit ci-dessus utilisant un transposon désarmé contenant un, plusieurs ou la totalité des gènes pour la biosynthèse des métabolites.

Parmi les gènes d'intérêt industriel, il faut citer les gènes codant pour la biosynthèse d'acides aminés notamment la thréonine, ou pour la biosynthèse de vitamine telle que la biotine. Mais l'amplification de l'expression de certaines enzymes peut permettre également une sur-expression globale de certains métabolites, ainsi les exemples montrent-ils la sur-expression du gène asd et du gène aspA.

Le procédé selon l'invention est très polyvalent et peut être utilisé pour amplifier tout type de séquence d'ADN.

La bactérie ayant la capacité de produire une ou plusieurs enzymes, ou un ou plusieurs acides aminés ou des produits voulus, ainsi obtenue peut être cultivée de manière quasi habituelle. On emploie des milieux de culture ordinaires contenant des sources de carbone, d'azote, des ions inorganiques et, si nécessaire, des micronutriments organiques tels que des acides aminés et des vitamines. Comme source de carbone convenable, on trouve le glucose, le sucrose, le lactose, l'hydrolysat d'amidon contenant ces sucres, le petit lait et la mélasse. Parmi les sources d'azote, on préfère l'ammoniac, l'ammoniaque et les sels d'ammonium. La culture est menée dans des conditions aérobiques, sous pH et température contrôlés jusqu'à ce que les cellules cessent pratiquement de produire le produit voulu.

Pour une production telle que celle d'un acide aminé ou d'une protéine, la souche qui produit déjà le produit voulu est préférable comme souche hôte pour la production envisagée.

Les exemples suivants ainsi que les figures illustreront d'autres caractéristiques et avantages de la présente invention.
- Figure 1 : Représente schématiquement la carte de restriction du transposon MudII1734 à large spectre d'hôtes.
- Figure 2 : Représente schématiquement la construction et la carte de restriction des plasmides pBR322 (MudII1681) et pPR30.
- Figure 3 : Montre les résultats de l'électrophorèse suivie par la technique de "blot" hybridation de :
   . l'ADN d'une souche dans laquelle le nombre de copies de transposon a été amplifié (trace f),
   . l'ADN de 5 souches isolées après 170 générations (canaux a à e), (la sonde est pCE1134 ; l'ADN a été digéré par EcoRI).
- Figure 4 : Représente schématiquement la construction et la carte de restriction du plasmide pCE1134.
- Figure 5 : Représente schématiquement la construction et la carte de restriction du plasmide pAM9.
- Figure 6 : Montre les résultats de l'électrophorèse suivie par "blot" hybridation de l'ADN des souches AJ11332, EL1001, EL1002, et EL1003 respectivement pour les canaux A à D ; (la sonde est un petit fragment EcoRI de pAM9 ; l'ADN des souches a été digéré par EcoRI).
- Figure 7 : Représente schématiquement la construction de pEV11, pMC1 et pMC23.
- Figure 8 : Représente les analyses des transpositions de MudAM9 par la technique d'hybridation ADN-ADN de Southern dans les souches AJ11332, EL1008 et EL1009.
- Figure 9 : Représente les résultats de l'électrophorèse suivie par "blot" hybridation de l'ADN des souches AJ11332, EL1001, EL1004, EL1006, EL1003, EL1005 et EL1007, respectivement pour les canaux A à G (la sonde correspond au plasmide pRC100 digéré par HindIII).
- Figure 10 :Montre les résultats de l'électrophorèse suivie par "blot" hybridation de l'ADN des souches E. chrysanthemi 384551, EL3003 et EL3007 pour les canaux A, B et C respectivement (la sonde utilisée est le fragment EcorRI-HindIII du plasmide pAM9).
- Figure 11 :Montre les résultats d'électrophorèse suivie par "blot" hybridation de l'ADN des souches E. chrysanthemi 384551 EL3004 et EL3008 pour les canaux A à C respectivement (la sonde utilisée est le fragment SalI-BamHI du plasmide pAM9.
- Figure 12 :Montre la construction du plasmide pAM11 contenant le gène asd et l'opéron thréonine.
- Figures 13 à 16 : Montrent les résultats d'électrophorèse suivie par "blot" hybridation de l'ADN des souches EL1016, EL1012 et EL1013 pour les canaux A à C respectivement.
   Pour la figure 13, les ADN chromosomiques sont digérés par SalI et hybridés avec le fragment EcoRI-HindIII contenant les gènes nptII, thrA, thrB et thrC' de pAM9 comme sonde.
   Pour la figure 14, les ADN chromosomiques sont digérés par EcoRI et hybridés avec le fragment EcoRI-HindIII, contenant les gènes nptII, thrA, thrB et thrC' de pAM9 comme sonde.
   Pour la figure 15, les ADN chromosomiques sont digérés par SalI et hybridés avec le fragment BamHI, contenant le gène asd, de pAM11 comme sonde.
   Pour la figure 16, les ADN chromosomiques sont digérés par EcoRI et hybridés avec le fragment BamHI, contenant le gène asd, de pAM11 comme sonde.

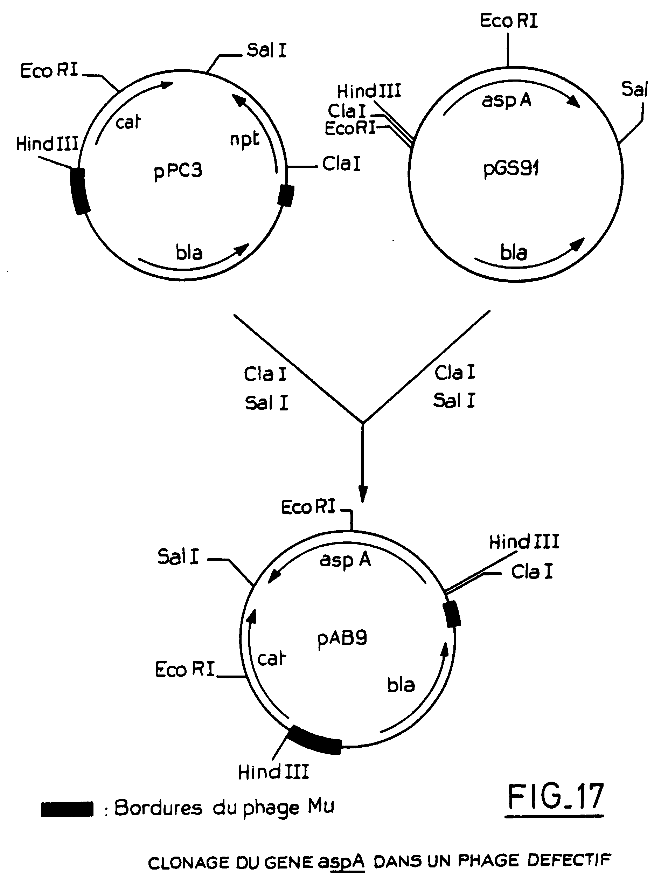

- Figure 17 :Représente le clonage du gène aspA dans un phage défectif.

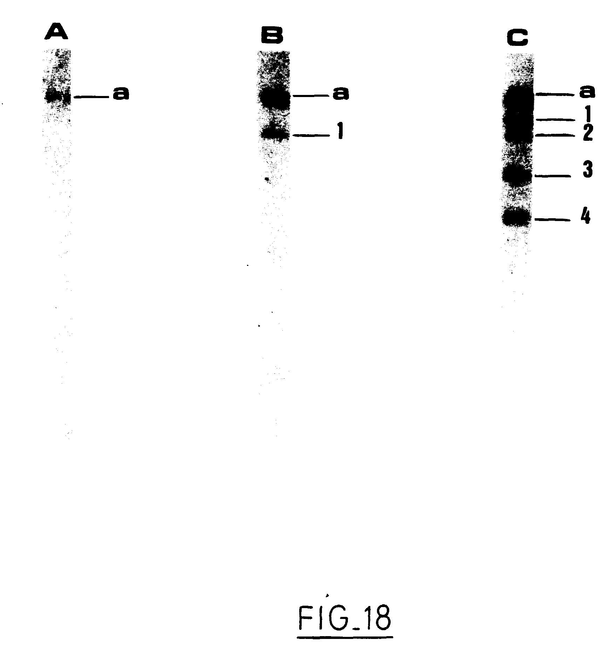

- Figure 18 :Montre les résultats d'électrophorèse suivie par "blot" hybridation de l'ADN des souches MC4100, EL1010 et EL1011 pour les canaux A à C respectivement. La sonde est un plasmide contenant le gène aspA (l'ADN a été digéré par SalI).

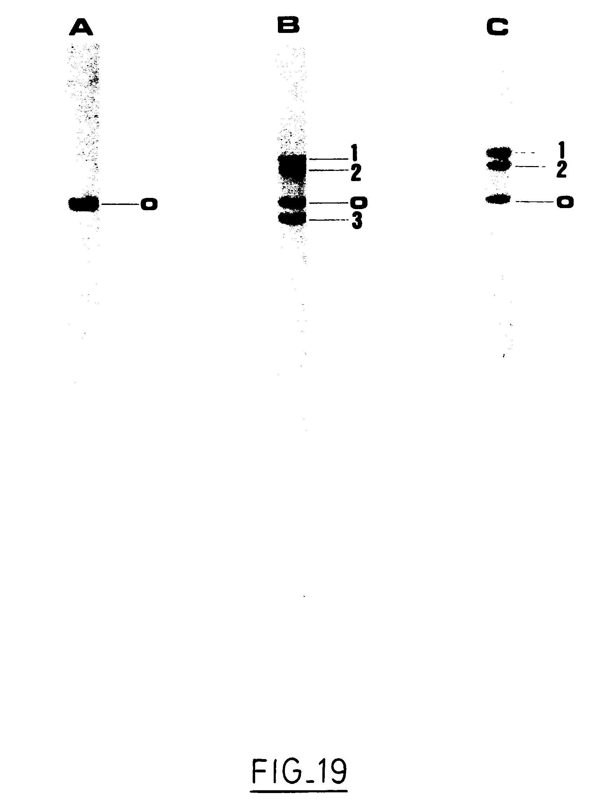

- Figure 19 :Montre les résultats d'électrophorèse suivie par "blot" hybridation de l'ADN des souches EL1016, EL1014, EL1015 pour les canaux A à C respectivement. La sonde est le fragment SalI-BamHI contenant l'opéron thréonine du plasmide pAM9, l'ADN est digéré par l'enzyme HindIII.

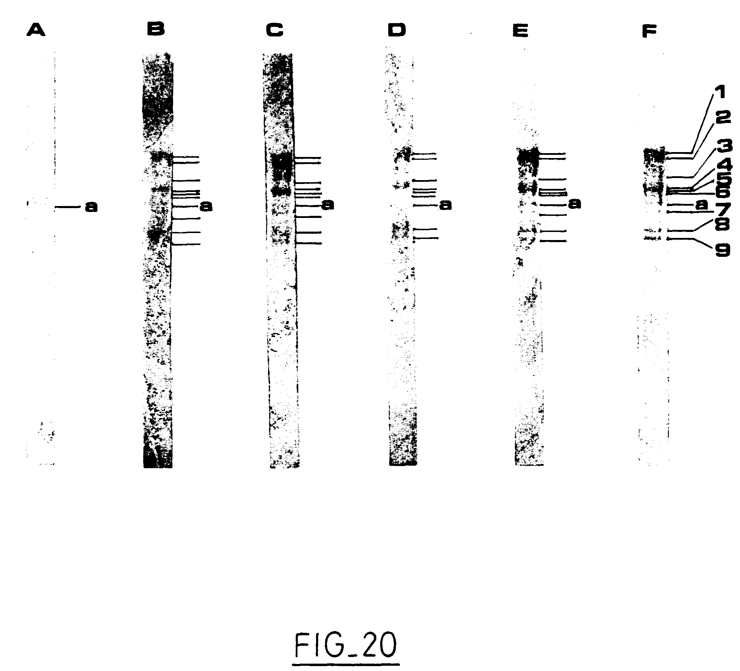

- Figure 20 :Représente les résultats d'électrophorèse suivie par "blot" d'hybridation de l'ADN des souches EL1016, EL1017, EL1017-a, EL1017-b, EL1017-c et EL1017-d pour les canaux A à F respectivement (la sonde est le fragment EcoRI-HindIII contenant les gènes nptII, thrA, thrB et thrC' du plasmide pAM9.

### EXEMPLE 1 : PRODUCTION D'UNE ENZYME AYANT UNE ACTIVITE BETA-GALACTOSIDASE PAR AMPLIFICATION DE LA REGION CODANTE RESPONSABLE DE LA PRODUCTION PAR FERMENTATION DE BETA-GALACTOSIDASE

### Préparation d'une souche contenant dans son chromosome une copie supplémentaire du gène Béta-galactosidase cloné à l'intérieur d'un transposon désarmé

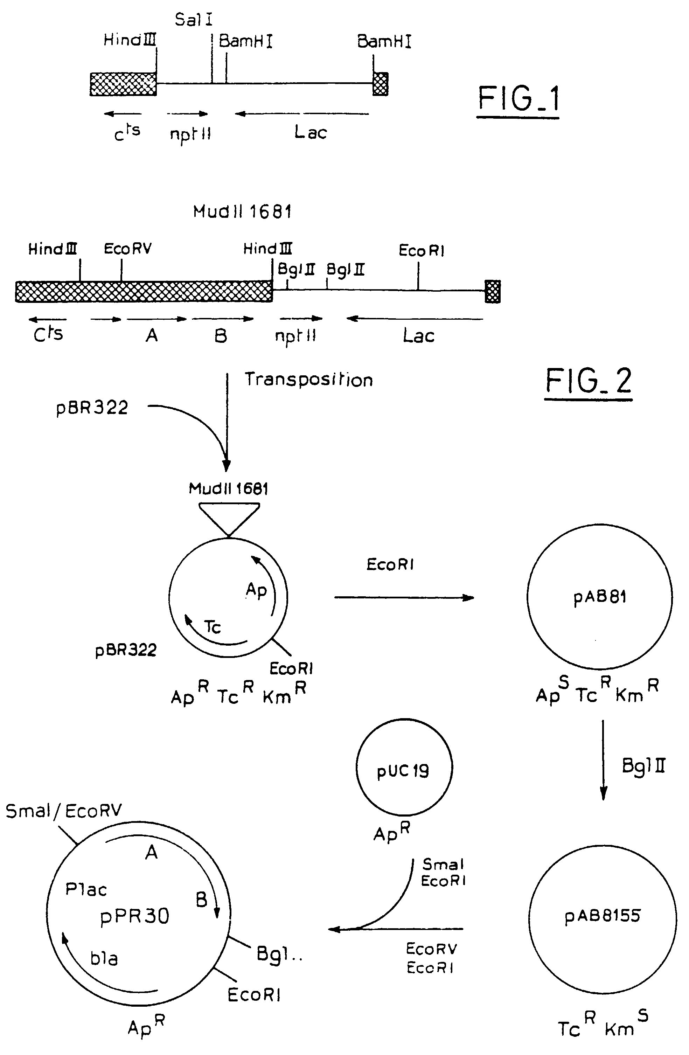

La souche d'E. coli POII1734 : F⁻, araD139, ara : (Mucts 3, Δ (lac)X74, galU, galK, rpsL (Sm^{R}), (MudII1734) contenant MudII1734 (Fig. 1) (Castilho et al., J. Bacteriol., 158, 488, 1984) a été cultivée dans 2 ml de milieu LB pendant 2 heures à 30°C, a ensuite subi un choc thermique à 45°C pendant 15 minutes puis a été cultivée à 37°C pendant 2 heures, ceci menant à un lysat de phage. Pour obtenir la solution de phage, ce lysat a été mélangé avec plusieurs gouttes de chloroforme et ensuite centrifugé. Le surnageant a été utilisé comme solution de phage pour infecter la souche de bactérie.

La souche d'E. coli MC4100 : F-araD139, Δ(argF-lac), U169, psL150, relAl, flb5301, ptsF25, deoCI (Casabadan, 1975) telle que décrite par Castilho et al. (1984) a été cultivée pendant une nuit sur milieu LB. On ajoute ensuite du CaCl₂ et du MgSO₄ jusqu'à une concentration finale de I mM et 2,5 mM respectivement. 200 µl de cette culture et de la solution de phage décrite ci-dessus sont mélangés et laissés à température ambiante pendant 15 minutes. Après addition de 2 ml de milieu LB et incubation pendant 2 heures à 30°C, cette suspension de cellules est étalée sur boîte de milieu LB contenant 20 µg/ml de kanamycine (Km) et 10 mM de X-gal et ensuite mise en incubation pendant 24 heures à 30°C pour sélectionner les transductants.

On identifie les colonies qui ne produisent pas de Mucts après vérification par étalement des répliques sur une souche d'E. coli sensible au Mu et on conserve une souche MC4100 (MudII1734). Cette souche ne produit pas de Béta-galactosidase comme le montre la couleur blanche des colonies sur les boîtes de milieu LB supplémenté en X-gal, une substance qui passe de l'incolore au bleu quand elle est hydrolysée par la Béta-galactosidase.

L'ADN est extrait, scindé avec l'enzyme de restriction EcoRI et les fragments obtenus séparés sur un gel et absorbés sur nitrocellulose. Après hybridation avec l'ADN du transposon marqué, deux bandes sont visibles sur l'autoradioagramme obtenu indiquant la présence d'une copie du transposon dans la souche.

### Clonage des gènes de transposase A et B du Mu phage sur un plasmide multicopie

L'ADN extrait du plasmide pAB8155 contenant le phage désarmé MudII1681 est scindé avec les enzymes de restriction EcoRI et EcoRV. Le fragment de 8,3 kb qui contient les gènes A et B de la transposase est purifié par électrophorèse sur gel et cloné dans le vecteur circulaire pUC19 linéarisé avec SmaI et EcoRI. Le plasmide reconstitué est transformé dans une bactérie réceptrice convenable. Les colonies résistant aux antibiotiques sont sélectionnées et on montre qu'elles contiennent le plasmide pPR30 (Fig. 2).

MC4100 (MudII1734) est transformé avec pPR30 après traitement des cellules au chlorure de calcium (Mandel et Higa, J. Mol. Biol., 53, 159, 1970). Après 2 heures à 30°C sur milieu LB les cellules sont étalées sur des boîtes contenant 20 µg/ml de Km, 25 µg/ml d'ampicilline (Ap) et 10 µg/ml de X-gal. On incube pendant 48 heures à 30°C et on voit apparaître des colonies colorées en bleu d'intensité variable. Ces colonies sont purifiées sur le même milieu et sélectionnées.

L'ADN total est extrait de colonies indépendantes, digéré avec EcoRI puis soumis à électrophorèse et transféré sur membrane de nylon. Après hybridation avec l'ADN du transposon radiomarqué, on détecte plusieurs bandes sur l'autoradiogramme mettant en évidence l'amplification du transposon. Le plasmide pPR30 est éliminé de ces souches par plusieurs transferts successifs sur milieu LB sans Ap.

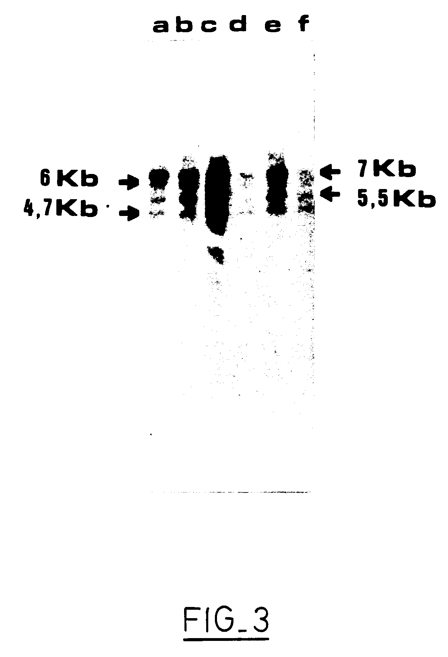

La souche AMI a été particulièrement étudiée : après que l'ADN ait été isolé, digéré par EcoRI et ait subi une électrophorèse, les fragments d'ADN sont transférés sur membrane de nylon. On peut voit 4 bandes d'hybridation sur la figure 3 (bande f) quant on utilise comme sonde un pCE1134 radiomarqué. Ces 4 bandes d'hybridation correspondent à deux insertions du MudII1734.

### Stabilité du nombre de copie de MudII1734

La souche AMI : MC4100 (MudII1734) qui a deux copies de MudII1734 sur son chromosome est cultivée pendant 170 générations sur milieu liquide LB sans Km. Puis le nombre de copie de MudII1734 sur le chromosome est mesuré par hybridation ADN-ADN (fig. 3, bandes a à e). Le nombre de copies dans cette souche est resté stable, aucun changement dans les schémas de restriction n'a pu être détecté, ce qui indique une grande stabilité des insertions.

### Production de beta-galactosidase

La souche ainsi obtenue est capable de produire de la Béta-galactosidase. Les souches MC4100 (MudII1734) et AM1 produisent respectivement 7.10⁻¹ et 10 nMole/mn/mg de protéine ayant une activité Béta-galactosidase.

### EXEMPLE 2 : PRODUCTION DE L-THREONINE PAR AMPLIFICATION DES GENES RESPONSABLES DE LA PRODUCTION DE L-THREONINE PAR FERMENTATION

### Construction de pCE1134

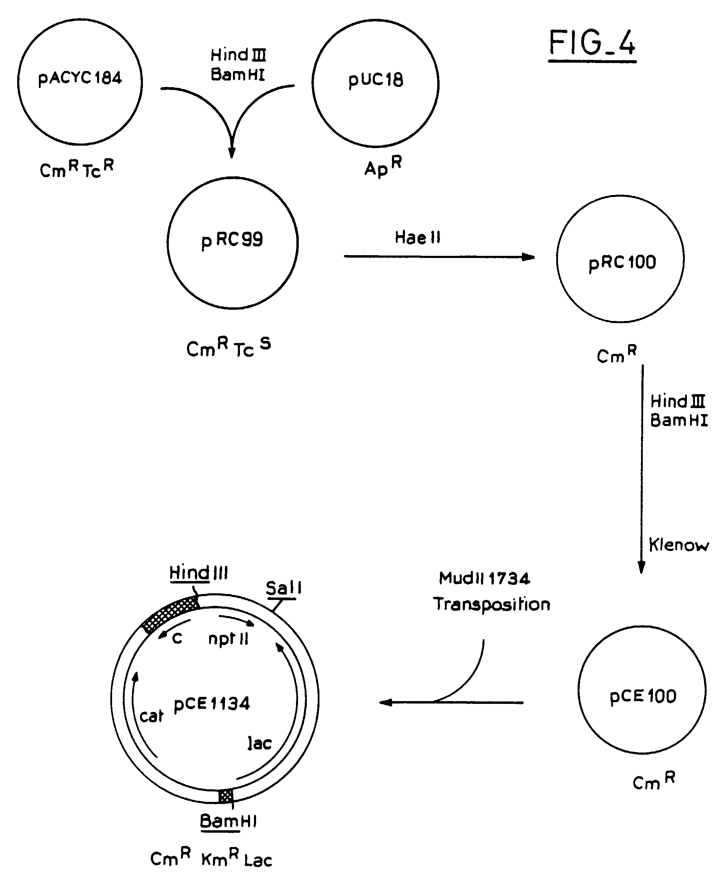

La souche POII1734 (Castilho et al., J. Bacteriol.. 158, 488, 1984) est transformée avec pCE100 (2,1 kb, Cm^{R}, Fig. 4) après traitement des cellules avec du chlorure de calcium (Mandel A. et Higa M., J. Mol. Biol., 53, (1970)).

Les transformants sont sélectionnés sur des boîtes de milieu LB contenant du 25 µg/ml de chloramphénicol (Cm) après 24 heures d'incubation à 30°C. Le transformant PO111734/pCE100 est cultivé pendant 2 heures à 30°C dans 3 ml de milieu LB, soumis ensuite à un choc thermique pendant 15 minutes à 45°C puis cultivé à 37°C pendant 2 heures, conduisant à un lysat de phage. Pour obtenir la solution de phage, ce lysat est mélangé avec plusieurs gouttes de chloroforme et ensuite centrifugé. Le surnageant est appelé solution de phage 1.

E. coli M8820 (Mu) (Castilho et al., J. Bacteriol., 158, 488, 1984) est cultivé pendant une nuit sur un milieu LB, auquel on ajoute du chlorure de calcium et du sulfate de magnésium à une concentration finale de I mM et 2,5 mM respectivement. 200 µl de cette culture et de la solution de phage I sont mélangés et laissés à température ambiante pendant 15 minutes. Après addition de 2 ml de milieu LB et incubation à 30°C pendant 2 heures, cette suspension de cellules est étalée sur des boîtes de milieu LB contenant 20 µg/ml de Km et 25 µg/ml de Cm, puis mise en incubation à 30°C pendant 24 heures. On récolte les 20 clones qui sont apparus sur les boîtes et on procède à une extraction de plasmide par la méthode de mini-préparation (Birnboim et Doly, Nucleic Acids Res., 7, 1513, 1979). L'analyse de la carte de restriction a montré que tous les clones présentaient un plasmide dans lequel MudII1734 était inséré. Un d'entre eux a été nommé pCE1134 (Fig. 4).

### Construction de MudAM9 comprenant l'opéron Thr

PCE1134 a été complètement clivé par les enzymes de restriction BamHI et ensuite SalI. On isole le fragment d'ADN, dans lequel l'opéron lac est manquant, par électrophorèse sur gel d'agarose et on l'extrait à partir de ce gel.

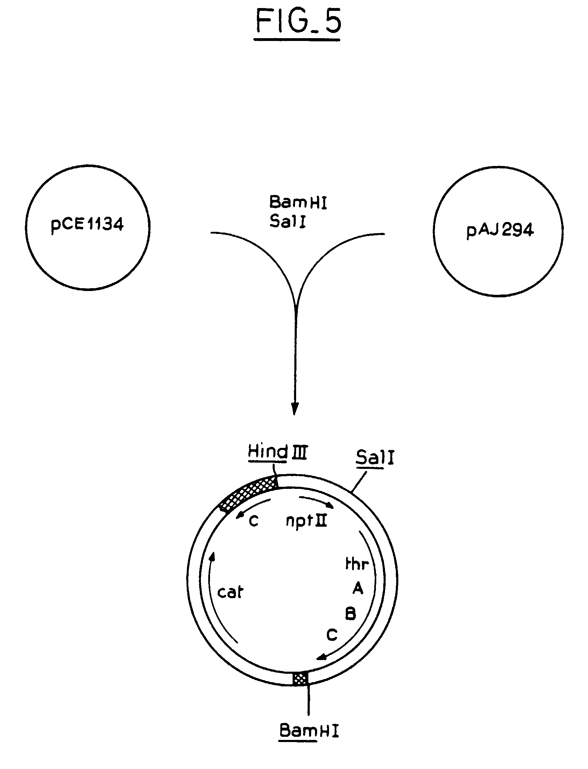

Le plasmide pAJ294 (Miwa et al., Agric. Biol. Chem., 47, 2329, 1983) a été complètement clivé par les enzymes de restriction BamHI puis SalI. Après une électrophorèse sur gel d'agarose, le fragment d'ADN qui contient l'opéron thr est extrait du gel. Les deux fragments d'ADN sont mélangés et on procède à la ligation à 10°C pendant 16 heures au moyen de T4-ADN ligase, d'ATP et de dithiothréitol. Après transformation de la souche mutante thrB avec le mélange de ligation, les transformants ont été sélectionnés sur un milieu minimum contenant 25 µg/ml de Cm et pas de thréonine. Le plasmide est extrait d'un des transformants et s'avère contenir Mud avec le gène npt et l'opéron thr (pAM9, Fig. 5).

### Transposition de MudAM9 sur l'ADN chromosomique

La souche d'E. coli JM109 (Yanisch-Perron et al., Gene, 33, 103-119 (1985)) (Mucts) est transformée avec pAM9 après traitement au chlorure de calcium. Les transformants sont sélectionnés sur des boîtes de milieu LB contenant 20 µg/ml de Km et 25 µg/ml de Cm, après incubation à 30°C pendant 24 heures. Les plasmides sont extraits de 20 colonies indépendantes et s'avèrent être pAM9. JM109 (Mucts)/pAM9 est cultivé à 30°C pendant 2 heures après avoir subi un choc thermique à 45°C pendant 15 minutes. On maintient la culture à 37°C pendant 2 heures. Ensuite, on mélange la culture avec quelques gouttes de chloroforme et on laisse à température ambiante pendant 15 minutes. On obtient la solution de phage II après 10 minutes de centrifugation à 5 000 tours/minute.

A la culture de JM109 (Mucts) laissée une nuit à 30°C sur milieu LB, on ajoute du CaCl₂ et du MgSO₄ jusqu'à une concentration finale de I mM et 2,5 mM, respectivement. 200 µl de cette solution et de la solution de phage Il sont mélangés et laissés à température ambiante pendant 15 minutes. Après addition de 2 ml de milieu LB, on laisse en incubation à 30°C pendant 2 heures et on étale cette suspension de cellules sur des boîtes de milieu LB contenant 20 µg/ml de Km. Après incubation à 30°C pendant 24 heures, on récolte 100 colonies et on vérifie leur sensibilité au Cm. 95 % d'entre elles sont sensibles au Cm et s'avèrent être des JM109 (Mucts) (MudAM9).

Après culture de JM109 (Mucts) (MudAM9) à 30°C pendant 2 heures sur milieu LB, on procède à un choc thermique pendant 15 minutes à 45°C et on poursuit par une incubation à 37°C pendant 2 heures. On ajoute ensuite quelques gouttes de chloroforme et on centrifuge à 5 000 tours/minute pendant 10 minutes . Le surnageant obtenu est la solution de phage III.

A la souche d'E. coli AJ11332 (Shiio et al., Agric. Biol. Chem., 33, 1152, 1969), cultivée pendant une nuit, on ajoute du CaCl₂ et du MgSO₄ jusqu'à des concentrations finales de 1 mM et de 2,5 mM, respectivement. On mélange 200 µl de cette solution et la solution de phage III, on laisse à température ambiante pendant 15 minutes. Après addition de 2 ml de milieu LB on continue l'incubation à 30°C pendant 2 heures. La suspension de cellules est étalée sur des boîtes de milieu LB contenant 20 µg/ml de Km et on met ensuite en incubation à 30°C pendant 24 heures. On récolte 100 colonies et on teste leur lyse à 45°C et leur sensibilité au phage Mu. 85 % des colonies ne lysent pas et sont sensibles à Mu. Une de ces souches est appelée EL1001.

### Amplification de MudAM9 sur l'ADN chromosomique et choix du nombre de copie

**(i)** Amplification de MudAM9 par pPR30 :
   On transforme la souche EL1001 avec pPR30. Les transformants sont sélectionnés sur des boîtes de milieu LB contenant 25 µg/ml d'Ap et une quantité variable de Km et/ou de Néomycine (Nm).
**(ii)** Amplification par lysat de Mucts :
   La souche EL1001 est infectée par un lysat de Mucts tel que décrit ci-dessus et ensuite étalée sur des boîtes de milieu LB contenant des quantités variables de Km.

Les clones obtenus à partir de pPR30 dans le cas de (i) et ceux obtenus à partir de Mu dans le cas de (ii) sont sélectionnés pour avoir intégré un nombre stable de copies de MudAM9.

Après incubation à 30°C pendant 48 heures, on récolte les colonies et on mesure le nombre de MudAM9 sur l'ADN chromosomique par la méthode d'hybridation décrite ci-dessus.

On étudiera plus particulièrement les souches EL1002 et EL1003.

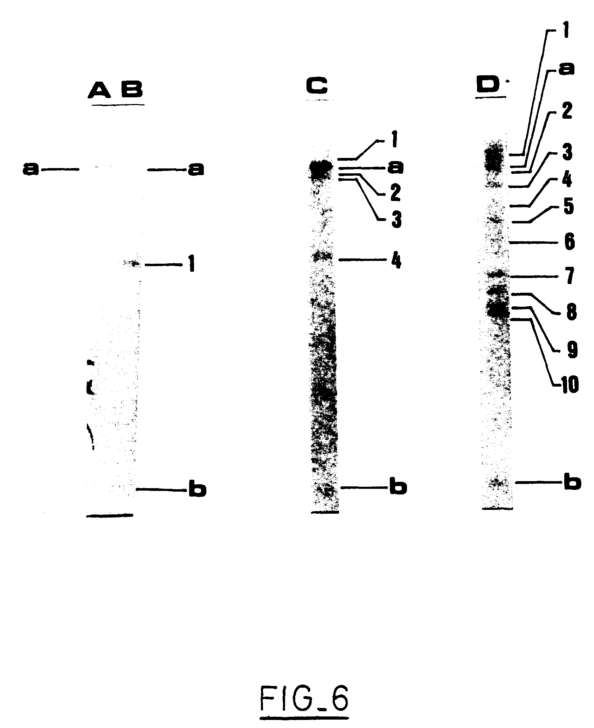

L'ADN est extrait des souches AJ11332, EL1001, EL1002 et EL1003. Après digestion par l'enzyme de restriction EcoRI, on soumet l'ADN à une électrophorèse et on le transfère sur membrane de nylon. L'ADN est ensuite hybridé avec des petits fragments de pAM9 radiomarqués digérés par EcoRI. Les résultats sont présentés sur la figure 6. Les bandes A, B, C et D correspondent respectivement aux souches AJ11332, EL1001, EL1002 et EL1003. La lettre a correspond au type sauvage de l'opéron thr, la lettre b correspond à une bande non identifiée que l'on voit sur les bandes B, C et D. Les chiffres correspondent aux bandes spécifiques de MudAM9. Selon ces résultats, les souches EL1001, EL1002 et EL1003 ont 1, 4 et 10 copies (au moins) de MudAM9.

La relation entre le nombre de copie de MudAM9 dans la souche et la concentration d'antibiotiques auxquels la souche est résistante est résumée dans le tableau I :

**TABLEAU I**

| **Relation entre le nombre de copies de MudAM9 et la résistance à l'antibiotique** | | |
|---|---|---|
| Nbre de copies de MudAM9 | Résistance | Sensibilité |
| 0 | | 20 µg/ml Km |
| 1 | 200 µg/ml Km | 250 µg/ml de Km et Nm |
| 2 (ou plus) | 250 µg/ml de Km et Nm | |

### Production de L-thréonine

Les souches ayant MudAM9 amplifié sont repiquées sur boîtes de milieu LB à 30°C pendant 24 heures et inoculées dans 20 ml du milieu de production décrit ci-dessous.

### Composition du milieu de production

Glucose : 30 g/l, (NH₄)₂ SO₄ : 10 g/l, KH₂ PO₄ : 1 g/l, MgSO₄ 7H₂O : 1 g/l, FeSO₄ 7H₂O : 10 mg/l, MnSO₄ 4-6H₂O : 10 mg/l, L-Met : 0,1 g/l, L-Ile : 0,1 g/l, L-Pro : 0,45 g/l, ARN : 2 g/l, Thiamine HCl : 1 mg/l et CaCO₃ : 40 g/l (pH : 7,0).

La culture est agitée à 30°C pendant 72 heures. La quantité de thréonine dans le milieu est mesurée grâce à un analyseur d'acide aminé. Les résultats sont rassemblés dans le tableau II :

**TABLEAU II**

| **Production de L-Thréonine par des souches contenant MudAM9** | | |
|---|---|---|
| Souche | Estimation du nombre de copie de MudAM9 | L-Thr (g/l) |
| AJ11332 | 0 | 3 |
| EL1001 | 1 (au moins) | 3,3 |
| EL1002 | 4 (au moins) | 8,9 |
| EL1003 | 10 (au moins) | 12 |

### Stabilité et productivité en L-thréonine

La souche EL1002 contenant 4 copies de MudAM9 est transférée plusieurs fois successives sur boîtes de milieu LB sans antibiotiques et après chaque transfert on teste la productivité en L-thréonine en appliquant la méthode décrite ci-dessus. Les résultats sont rassemblés dans le tableau III :

**TABLEAU III**

| **Stabilité de la production de L-thréonine après transferts répétés sur milieux LB sans antibiotiques avec la souche EL1002** | | | | | | |
|---|---|---|---|---|---|---|
| Nombre de transferts | 0 | 2 | 4 | 6 | 8 | 10 |
| Production de L-Thr | 8,5 | 8,6 | 8,5 | 8,8 | 8,8 | 8,9 |

Le tableau IV montre les activités spécifiques des enzymes de la biosynthèse de L-Thr des souches contenant MudAM9 :

**TABLEAU IV**

| **Production en grande quantité des enzymes de la biosynthèse de la L-thréonine par AJ11332 (mudAM9)** | | |
|---|---|---|
| Souche | Activités spécifiques (*) | |
| | AKI | HDHI |
| AJ11332 | 2,5 | 4,8 |
| EL1001 | 4,0 | 10,1 |
| EL1002 | 12,5 | 25,0 |
| EL1003 | 22,5 | 37,4 |

| | | |
|---|---|---|
| (*) Les activités spécifiques sont données en µmole/min/mg de protéine | | |

### EXEMPLE 3 : AMPLIFICATION DE MUD AVEC UN PLASMIDE CONTENANT DES TRANSPOSASES THERMO-INDUCTIBLES

### Construction d'un plasmide contenant des transposases thermo-inductibles

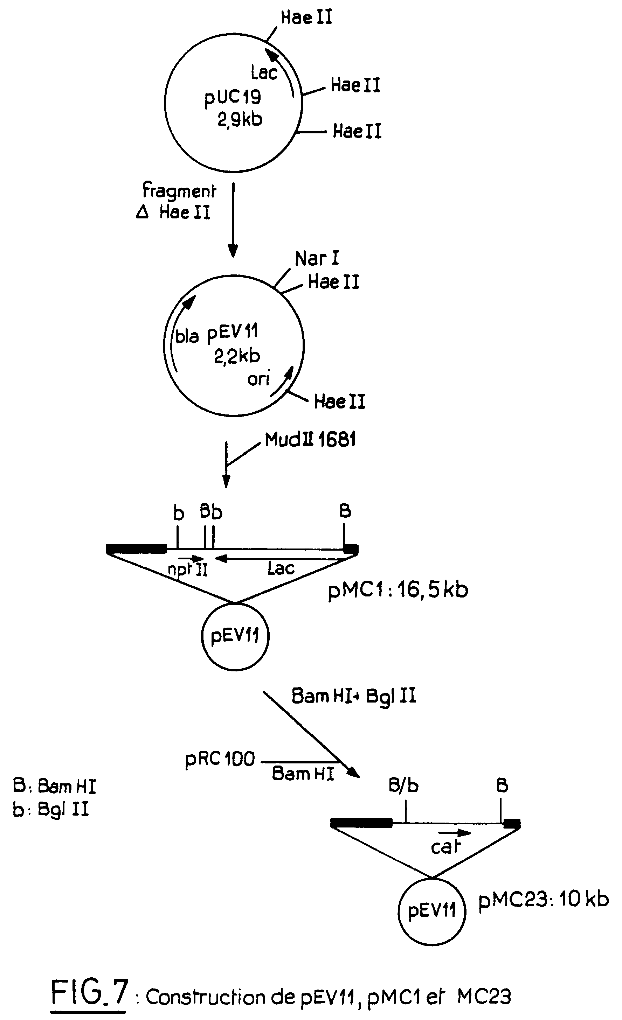

La souche POII1681 : M8820 (Mucts) (MudII1681) (Castilho et al., J., Bacteriol., 158, 488 (1984)) est transformée après traitement des bactéries au CaCl₂ (Mandel M. and Higa A., J. Mol. Biol., 53, 159 (1970)), avec le plasmide pEV11 (Fig. 7). Les transformants, après 24 Heures d'incubation à 30°C, sont sélectionnés sur boites de milieu LB contenant 20 µg/ml d'Ap et 20 µg/ml de Km. Un transformant POII1681/pEV11 est cultivé 2 heures à 30°C dans 3 ml de LB. La culture subit ensuite un choc thermique de 15 minutes à 45°C suivi de 2 heures de culture à 37°C. Quelques gouttes de chloroforme sont ajoutées, après une centrifugation de 10 minutes à 5000 tpm la solution de phages est obtenue. Une culture de nuit de la souche E. Coli : M8820 (Mu) (Castilho et al., J., Bacteriol., 158, 488, (1984) est réalisée en milieu LB ; du CaCl₂ et du MgSO₄ y sont ajoutés aux concentrations finales de 1 mM et 2,5 mM respectivement. A 200 µl de cette culture 50 µl de la solution de phages sont ajoutés, cette suspension est laissée à température ambiante 15 minutes.

Après avoir ajouté 2 ml de LB et agité 2 heures à 30°C, on étale la suspension de bactéries sur boites de milieu LB contenant 20 µg/ml d'Ap et 20µg/ml de Km ; une incubation de 24 Heures à 30°C est réalisée. Vingt colonies sont obtenues et l'on extrait leurs ADN plasmidiques par la méthode de mini-préparation (Birnboim et Doly, Nucleic Acid Res. 7, 1513 (1979)). Après analyses de la carte de restriction, les clones s'avèrent contenir une insertion du MudII1681 dans le plasmide pEV11. L'un des plasmides est appelé pMC1 (Fig. 7).

Le plasmide pMC1 est digéré par les enzymes de restriction BamHI et BglII puis ligué avec pRC100 (voir exemple 2) coupé par BamHI. La souche MC1060 est transformée par le mélange de ligation. Les transformants sont sélectionnés sur boites de milieu LB contenant 25 µg/ml de Cm et 20 µg/ml d'Ap. Les plasmides de 30 transformants, sont extraits et analysés ; l'un d'entre eux est appelé pMC23 (Fig. 7).

### Amplification du phage défectif MudAM9 avec le plasmide pMC23

La souche EL1001 contenant le Phage défectif MudAM9 (voir exemple 2) est transformée, après traitement au CaCl₂ (exemple 2) par le plasmide pMC23. Les transformants sont sélectionnés sur boites de milieu LB contenant 20 µg/ml d'Ap et 25 µg/ml de Cm après une incubation de 24 heures à 30°C.

Un transformant EL1001/pMC23 est cultivé en milieu LB contenant 20 µg/ml d'AP et 25 µg/ml de Cm, 2 heures à 30°C. 1 ml de cette culture subit un choc thermique de 15 minutes à 45°C, puis 1 ml de LB est ajouté ; l'incubation a lieu 2 heures à 30°C.

Cette suspension bactérienne est étalée sur boites de milieu LB contenant 400 µg/ml de Nm et Km ; l'incubation est de 48 heures à 30°C. 50 clones apparus, sont testés pour leur sensibilité au Cm et leur production de L-Thr (exemple 2). 50% parmi eux sont Cm sensibles et 60% produisent plus de L-Thréonine que la souche hôte.

Plusieurs clones sont ainsi sélectionnés, et leur nombre de copie de MudAM9 est mesuré par la technique d'hybridation ADN-ADN (Southern, 1975, J. Mol. Biol., 98 503). Les ADN chromosomiques sont digérés par l'enzyme de restriction HindIII. Après électrophorèse, les ADN sont absorbés sur une membrane de nylon puis hybridés avec le fragment (marqué par sulfonation) SalI-BamHI, contenant l'opéron thréonine, du plasmide pAM9 comme sonde.

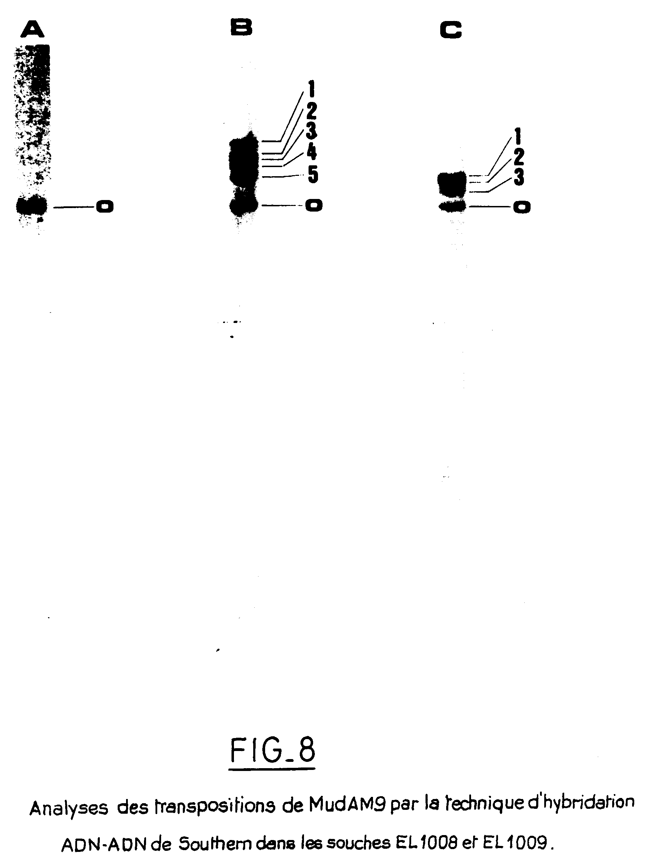

Les résultats concernant la production de L-Thréonine et le nombre de copies de MudAM9 sont consignés dans le Tableau V et la figure 8. Les canaux A, B et C correspondent respectivement aux souches AJ11332, EL1008 et EL1009.

Dans le canal A, la bande "o" correspondant à l'opéron thréonine est révélée. Dans le canal B, 6 bandes au moins sont révélées : la bande "o" et 5 bandes qui sont numérotées 1 à 5 et qui correspondent à 5 copies (au moins) du MudAM9. Dans le canal C, en plus de la bande "o", 3 bandes apparaissent numérotées 1 à 3 qui correspondent à 3 copies au moins du MudAM9.

**TABLEAU V**

| **Nombre de copies du MudAM9 et production de L-Thréonine dans les souches EL1008 et EL1009** | | |
|---|---|---|
| Souche | Nombre de copies | L-Thr(g/l) |
| EL1008 | 5 | 7 |
| EL1009 | 3 | 5 |

### EXEMPLE 4: AMPLIFICATION DE DEUX PHAGES DEFECTIFS DIFFERENTS SUR L'ADN CHROMOSOMIQUE D'UNE SOUCHE

### Préparation d'un lysat contenant le MudIIPR13

Après culture de la souche MC4100 (Mucts) (MudIIPR13) (P. Ratet et al., Gene, 63, 41-52, 1988) en milieu LB pendant 2 heures à 30°C, un choc thermique de 15 min à 45°C est effectué suivi d'une incubation à 37°C pendant 2 heures. Quelques gouttes de chloroforme sont ajoutées puis une centrifugation de 10 min à 5000 tpm est effectuée. Le lysat de phage VI est ainsi obtenu.

### Transposition du MudIIPR13 sur l'ADN chromosomique

A une culture de nuit de la souche EL1001, du CaCl₂ et du MgSO₄ sont ajoutés jusqu'aux concentrations finales de 1 mM et 2,5 mM respectivement. 50µl de la solution de phage VI sont ajoutés à 200µl de la culture de EL1001 puis la suspension est placée pendant 15 Min à température ambiante. Après addition de 2 ml de LB, la suspension est agitée à 30°C pendant 2 heures. Les bactéries sont ensuite étalées sur un milieu LB contenant 25 µg/ml de Cm et 20 µg/ml de Km. Après une incubation de 24 heures à 30°C, 50 colonies sont repiquées puis testées pour leur lyse à 45°C ainsi que pour leur sensibilité au phage Mu. 80 % des colonies testées sont sensibles à Mu et ne lysent pas à 45°C. Une souche EL1001 (MudIIPR13) est appelée EL1004.

La souche EL1003 est traitée de la même façon.

Une souche EL1003 (MudIIPR13) est appelée EL1005.

### Amplification de MudIIPR13 dans les souches EL1004 et EL1005

La souche EL1004 est infectée par un lysat de Mucts préparé à partir de la souche JM109 (Mucts) par la méthode décrite dans l'exemple 2. La suspension bactérienne est étalée sur des boites de milieu LB contenant 20 µg/ml de Km et 400 µg/ml de Cm. Après incubation de 48 heures à 30°C, 50 colonies sont isolées puis testées pour leur lyse à 45°C ainsi que leur sensibilité au phage Mu. 84 % des clones sont sensibles à Mu et ne lysent pas à 45°C. Un de ces clones est appelé EL1006.

La souche EL1005 est traitée de la même façon et le clone EL1007 est obtenu.

Les ADN chromosomiques sont extraits des souches AJ11332, EL1001, EL1003, EL1004,EL1005, EL1006, et EL1007.

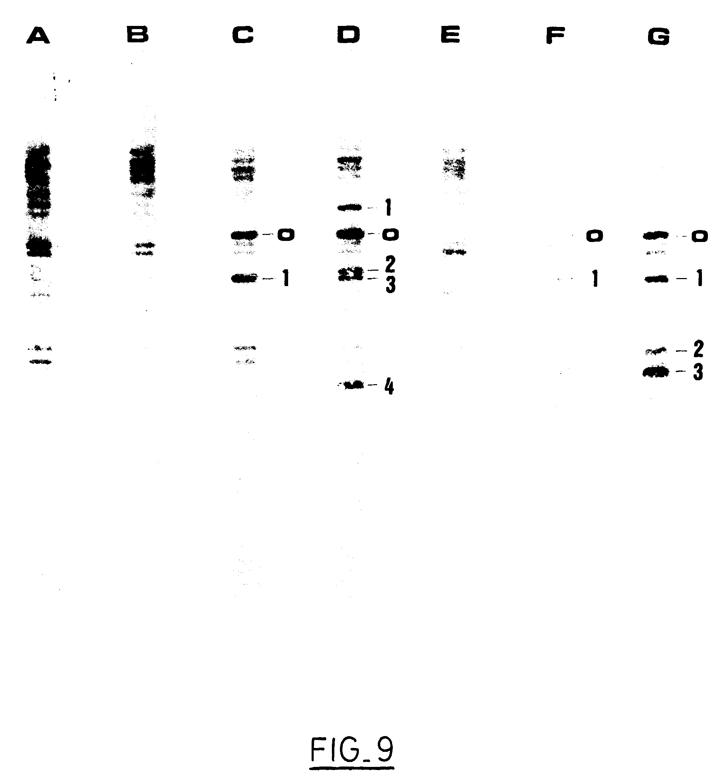

Après digestion des ADN chromosomiques par l'enzyme de restriction EcoRI, une hybridation ADN-ADN est effectuée par la méthode de Southern (exemple 3). La sonde correspond au plasmide pRC100 digéré par HindIII et marqué par sulfonation. Sur la figure 9, les canaux A, B, C, D, E, F et G correspondent respectivement aux souches AJ11332, EK1001, EL1004, EL1006, EL1003, EL1005 et EL1007.

Les bandes obtenues dans les canaux A, B et E correspondent à l'hybridation entre la sonde pRC100 et les ADN chromosomiques des souches. Ces bandes sont présentes dans tous les autres canaux et ne sont donc pas spécifiques du MudIIPR13. Dans les canaux C, D, F et G, les nombres 1 à 4 marquent les bandes présentant une spécificité d'hybridation avec MudIIPR13 chaque bande correspond à une insertion du MudIIPR13. Dans cs mêmes canaux, la bande "o" correspond à une bande interne au MudIIPR13. Selon ces résultats, les souches EL1004 et EL1005 contiennent 1 insertion du MudIIPR13 ; les souches EL 1006 et EL1007 contiennent respectivement 4 et 3 insertions du MudIIPR13, au moins.

### EXEMPLE 5 : INTEGRATION ET AMPLIFICATION DE PHAGES DEFECTIFS DANS UNE SOUCHE D'ERWINIA CHRYSANTHEMI

### Préparation des lysats de MudII1734 et MudAM9

Deux lysats de phages contenant les MudII1734 et MudAM9 sont préparés à partir des souches : POII1734 (Castilho et al., J. Bacteriol., 158, 488 (1984)) et JM109 (Mucts) (MudAM9) (exemple 2) selon la méthode décrite dans l'exemple 2.

### Transposition des phages Mud sur l'ADN chromosomique de Erwinia Chrysanthemi

La souche d'E. chrysanthemi 384551 (M. Chippaux, CNRS, Marseille) est utilisée comme souche réceptrice. La transduction (exemple 2) est effectuée à l'aide des 2 lysats (exemple 2). Les colonies capables de croître sur un milieu contenant 20 µg/ml de Km sont isolées puis testées pour leur lyse à 45°C ainsi que pour leur sensibilité au phage Mu. 70 % et 80 % des transductants obtenus après infection par les lysats MudII1734 et MudAM9 sont respectivement sensibles au phage Mu et ne sont pas lysés à 45°C. Un clone de chaque transduction est sélectionné :
EL3003 : E. chrysanthemi (MudII1734)
EL3004 : E. chrysanthemi (MudAM9)

L'amplification des phages Mud dans les souches EL3003 et EL3004 est effectuée à l'aide d'un lysat de la souche JM109 (Mucts) comme décrit dans l'exemple 2. La sélection des souches contenant les Mud amplifiés se fait sur un milieu LB contenant 400 µg/ml de Km et de Nm.

50 colonies résistantes sont repiquées puis testées pour leur sensibilité en phage Mu. Les souches EL3007 et EL3008 sensibles au phage Mu sont ainsi obtenues.

### Nombre de copies de Mud sur l'ADN chromosomique des deux souches

L'ADN chromosomique est extrait par la même technique que pour E. coli (exemple 1) excepté un traitement au SDS à la place du sarcosyl. Une analyse moléculaire de ces ADN est effectuée selon la méthode de Southern (exemple 3). Les ADN sont digérés par l'enzyme de restriction EcoRI. Les sondes utilisées sont spécifiques des phages défectifs et sont marquées par sulfonation :
Pour MudII1734 : Fragment EcoRI-HindIII du plasmide pAM9 correspondant à la bordure gauche du phage défectif.
Pour MudAM9 : Fragment SalI-BamHI du plasmide pAM9 correspondant à l'opéron thréonine d'E.coli.

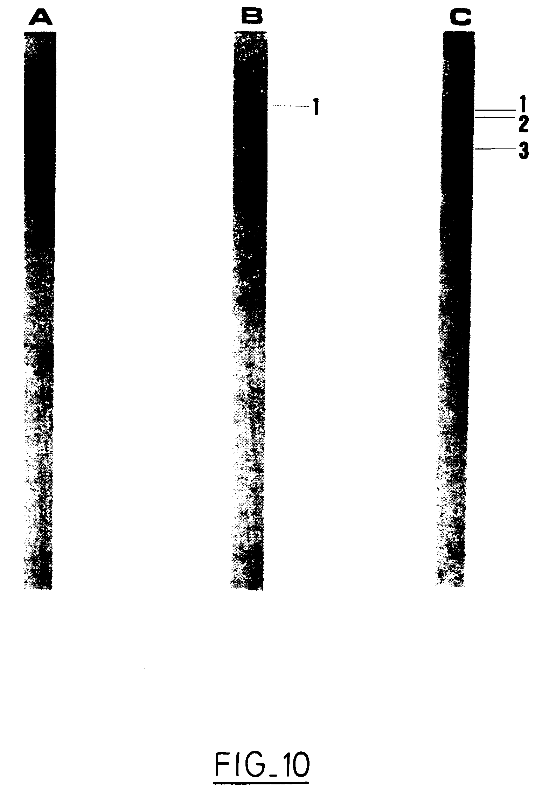

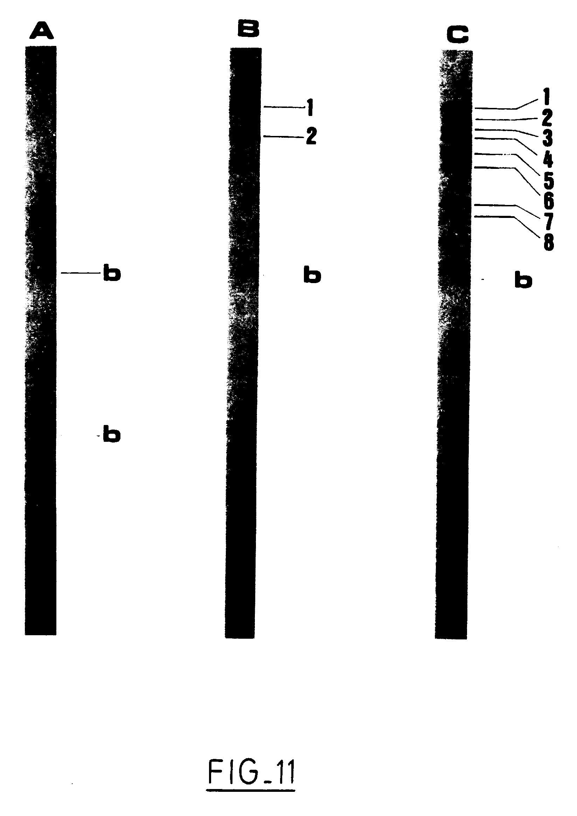

Les résultats sont présentés sur le tableau VI et les figures 10 et 11.

Sur la figure 10, les canaux A, B et C correspondent respectivement aux souches : E.chrysanthemi 384551, EL3003 et EL3007.

Dans les canaux B et C, 1 et 3 bandes apparaissent correspondant à une spécificité d'hybridation avec le MudII1734. Selon ces résultats, les souches EL3003 et EL3007 possèdent respectivement 1 et 3 insertions (au moins) de MudII1734.

Sur la figure 11, les canaux A, B et C correspondent respectivement aux souches : E. chrysanthemi 384551, EL3004, et EL3008.

Dans le canal A, le fragment "b" correspond à une hybridation entre la sonde et l'ADN chromosomique de la souche 384551. Ce fragment est retrouvé dans tous les autres canaux.

Dans les canaux B et C, les nombres correspondent à des bandes présentant une spécificité d'hybridation avec le MudAM9. Deux bandes correspondent à une insertion du MudAM9.

Selon ces résultats, les souches EL3004 et EL3008 possèdent respectivement 1 et 4 insertions au moins de MudAM9.

**TABLEAU VI**

| **Nombre de copies des Mud dans les souches d'E. chrysanthemi** | |
|---|---|
| souches | nombre de copies |
| EL3003 | 1 |
| EL3004 | 1 |
| EL3007 | 3 (au moins) |
| EL3008 | 4 (au moins) |

### EXEMPLE 6 : AMPLIFICATION D'UN PHAGE DEFECTIF DE TAILLE SUPERIEURE

### Construction du MudAM11 comprenant l'opéron thréonine et le gène asd

Le plasmide pAM9 (voir exemple 2), contenant le phage défectif MudAM9, est complètement clivé par l'enzyme de restriction BamHI (site unique) ; pAM9 est ainsi linéarisé.

Le plasmide pAD20 (Haziza et al., EMBO, J., 1982, 3, 379-384) est complètement clivé par l'enzyme de restriction BamHI. Après électrophorèse, le fragment qui contient le gène asd est extrait du gel.

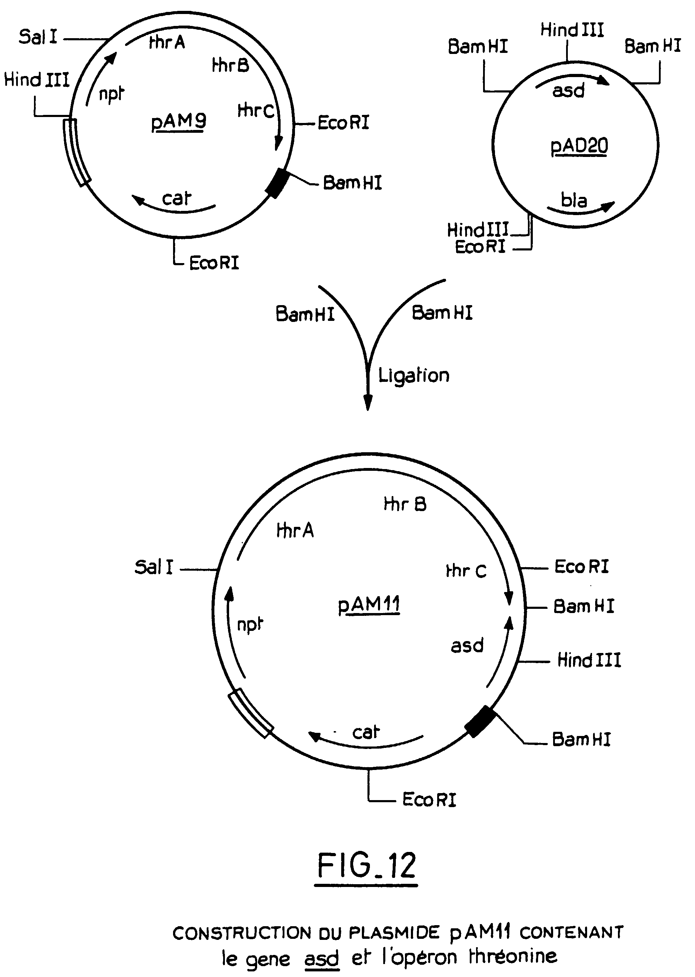

Les deux fragments d'ADN sont mélangés et l'on procède à une ligation à 22°C pendant 1 heure, au moyen de T4-ADN ligase, d'ATP et de dithiothréitol. Après transformation d'une souche mutante Asd⁻ : JCP203 (C. PRINTZ, communication personelle) avec le mélange de ligation, les transformants sont sélectionnés sur un milieu complet contenant 25 µg/ml de Cm et 20 µg/ml de Km. Un plasmide extrait des transformants s'avère présenter le phage défectif MudAM11 qui contient le gène nptII, l'opéron thréonine et le gène asd. Ce plasmide nommé pAM11, est présenté figure 12.

### Transposition du MudAM11 sur des ADN chromosomiques

La souche JM109 (Mucts)/pAM11 est obtenue par transformation de la souche d'E. coli JM109 (Mucts) (voir exemple 2, page 14, lignes 9 à 14), par le plasmide pAM11.

La souche JM109 (Mucts)/pAM11, est cultivée à 30°C pendant 2 heures. Après avoir subi un choc thermique à 45°C pendant 15 minutes, on maintient les cultures à 37°C pendant 2 heures. Ensuite, on additionne à cette culture quelques gouttes de chloroforme et on laisse à température ambiante pendant 15 minutes. On obtient la solution de phage II-AM11 après 10 minutes de centrifugation à 5000 tours/minute.

A la culture de JM109 (Mucts) laissée une nuit à 30°C en milieu LB, sont ajoutés de CaCl₂ et du MgSO₄jusqu'aux concentrations finales de 1 mM et 2,5 mM respectivement. 50 µl de la solution de phage II-AM11 sont mélangés à 200 µl de cette culture et laissés à température ambiante pendant 15 minutes. après addition de 2 ml de milieu LB, l'agitation est de 2 heures à 30°C puis on étale la suspension bactérienne sur boites de milieu LB contenant 20 µg/ml de Km. après incubation à 30°C pendant 24 heures, on récolte 100 colonies et on vérifie leur sensibilité au Cm, 85 % d'entre elles le sont, un clone est appelé JM109 (Mucts) (MudAM11).

Après culture de la souche JM109 (Mucts) (MudAM11), 2 heures à 30°C en milieu LB, on procède à un choc thermique de 15 minutes à 45°C. Après incubation de 2 heures à 37°C, on ajoute quelques gouttes de chloroforme et on centrifuge à 5000 tours par minute pendant 10 minutes.

Le surnageant obtenu est la solution de phage III-AM11.

La souche AJ11332 (λ⁺, Pro⁻) a été transduite par un phage P1 cultivé sur une souche Pro⁺. Une souche AJ11332 (λ⁺, Pro⁺) a été sélectionnée. Le phage λ contenu dans cette souche, a ensuite été éliminé (C. MOREL, communication personnelle). Le souche ainsi obtenue : AJ11332 (λ⁻, Pro⁺) a été appelée EL1016. A la souche d'E. coli EL1016, cultivée pendant une nuit à 37°C en LB, on ajoute du Ca Cl₂ et du MgSO₄ jusqu'aux concentrations finales de 1 mM et 2,5 mM respectivement. 50 µl de la solution de phage III-AM11 sont mélangés à 200 µl de cette culture et laissés à température ambiante 15 minutes. Après addition de 2 ml de LB, l'agitation est de 2 heures à 30°C. La suspension bactérienne est étalée sur boites de milieu LB contenant 20 µg/ml de Km, puis l'incubation est de 24 heures à 30°C. Un clone Mu sensible est sélectionné : EL1012.

### Amplification de MudAM11 sur des ADN chromosomiques et choix du nombre de copies

L'amplification se fait par infection de la souche EL1012 par un lysat de phages Mucts obtenu comme ci-dessus. Les bactéries sont étalées sur boites de milieu LB contenant des quantités variable de Km et Nm allant de 250 à 1200 µg/ml.

Les transductants sélectionnés contiennent un nombre stable de copies de MudAM11.

On étudie plus particulièrement la souche EL1013.

Le nombre de MudAM11 sur les ADN chromosomiques est évalué par la méthode d'hybridation ADN-ADN de Southern décrite dans l'exemple 3.

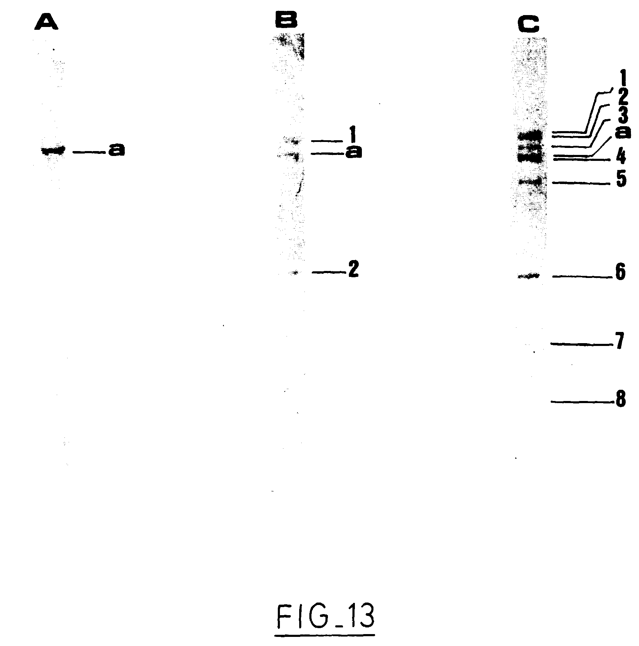

Les ADN chromosomiques sont extraits des souches EL1016, EL1012 et EL1013 respectivement. Après digestion par les enzymes de restriction SalI ou EcoRI, on soumet ces ADN à une électrophorèse puis on les absorbe sur membrane de nylon (voir exemple 3). Ils sont ensuite hybridés avec un fragment d'ADN marqué (par sulfonation) comme sonde (Figures 13, 14, 15 et 16), les canaux A, B et C correspondent respectivement aux souches EL1016, EL1012 et EL1013.

La lettre "a" correspond au type sauvage de l'opéron thréonine ou du gène asd. La lettre "p" correspond à des digestions partielles des ADN. Les nombres correspondent aux bandes marquant une spécificité d'hybridation avec MudAM11.

Pour la figure 13, les ADN chromosomiques sont digérés par SalI et hybridés avec le fragment EcoRI-HindIII contenant les gènes nptII, thrA, thrB et thrC', de pAM9 comme sonde.

Deux bandes correspondent dans ce cas à une insertion de MudAM11.

Dans le canal A, seule la bande "a" correspondant à l'opéron thréonine originel, est révélée.

Dans le canal B, trois bandes sont révélées : "a" et "1" et "2" qui correspondent à une insertion de MudAM11.

Dans le canal C, neuf bandes sont révélées : "a" et "1" à "8" qui correspondent à 4 insertions (au moins) de MudAM11.

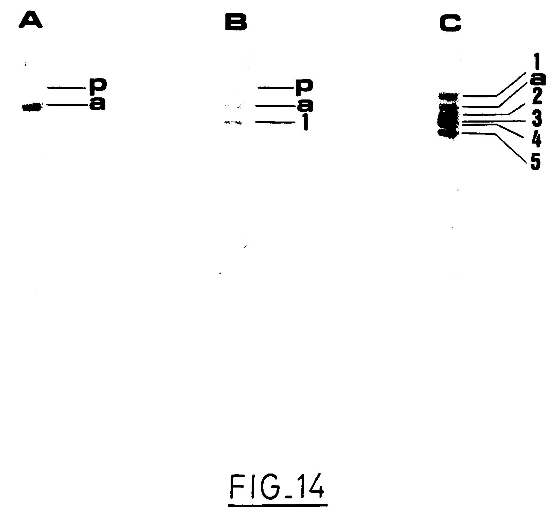

Pour la figure 14, les ADN chromosomiques sont digérés par EcoRI, et hybridés avec le fragment EcoRI-HindIII, contenant les gènes nptII, thrA, thrB et thrC' de pAM9 comme sonde. Une bande correspond à une insertion de MudAM11.

Dans le canal A, seule la bande "a" correspondant à l'opéron thréonine, est révélée.

La bande "p" correspond à une digestion partielle. Dans le canal B, deux bandes sont révélées : "a" et "1" correspondant à une insertion de MudAM11.

La bande "p" correspond à une digestion partielle.

Dans le canal C, six bandes sont révélées : "a" et "I" à "5" correspondant à 5 insertions (au moins) de MudAM11.

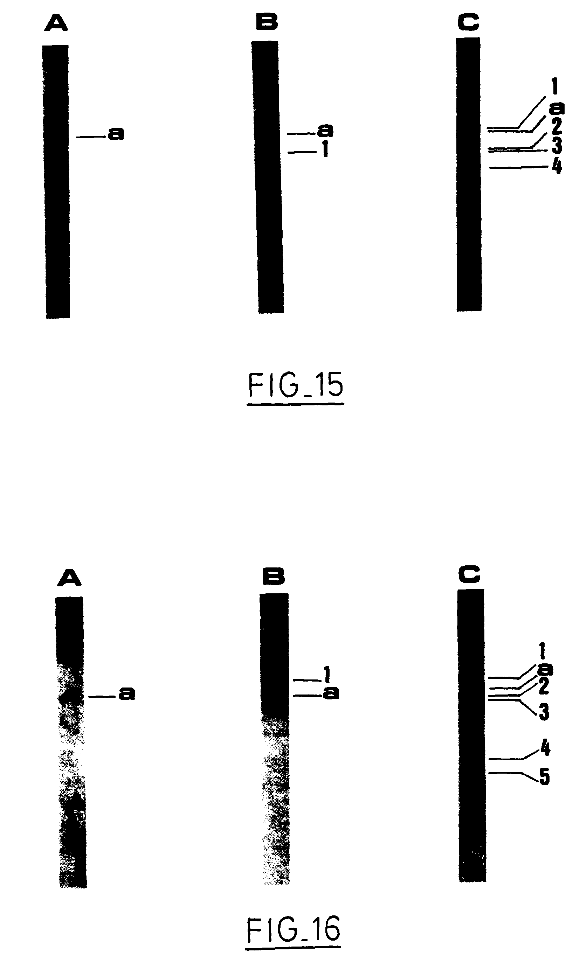

Pour la figure 15, les ADN chromosomiques sont digérés par SalI et hybridés avec le fragment BamHI, contenant le gène asd, de pAM11 comme sonde.

Une bande correspond alors à une insertion de MudAM11.

Dans le canal A, seule la bande "a" est révélée, correspondant au gène asd.

Dans le canal B, deux bandes sont révélées : "a" et "1" soit une insertion de MudAM11.

Dans le canal C, cinq bandes sont révélées : "a" et "1" à "4" qui correspondent à 4 insertions (au moins) de MudAM11.

Pour la figure 16, les ADN chromosomiques sont digérés par EcoRI et hybridés avec le fragment BamHI, contenant le gène asd, de pAM11 comme sonde.

Une bande correspond à une insertion de MudAM11.

Dans le canal A, seule la bande "a" correspondant au gène asd, est révélée.

Dans le canal B, deux bandes sont révélées : "a" et "1" soit une insertion de MudAM11.

Dans le canal C, six bandes sont révélées : "a" et "I" à "5" soit 5 insertions (au moins) de MudAM11.

Selon ces résultats, les souches EL1012 et EL1013 ont respectivement une et cinq copies (au moins) de MudAM11.

**TABLEAU VII**

| **Relation entre le nombre de copies de MudAM11 dans les souches, et les concentrations en antibiotiques auxquelles elles résistent** | | |
|---|---|---|
| Nombre de copies MudAM11 | Résistance | Sensibilité |
| 0 | | 20 µg/ml Km |
| 1 | 200 µg/ml Nm et Km | 250 µg/ml Nm et Km |
| 5 (ou plus) | 250 µg/ml Nm et Km | 1200 µg/ml Nm et Km |

**TABLEAU VIII**

| **Production de L-thréonine par les souches EL1012 et EL1013 contenant le phage défectif MudAM11** | | |
|---|---|---|
| Les souches ayant MudAM11 amplifié sont repiquées sur boites de milieu LB à 30°C pendant 24 heures, et inoculées dans 20 ml du milieu de production (exemple 2). La culture se fait à 30°C pendant 72 heures. La quantité de L-thréonine dans le milieu est mesurée grâce à un analyseur d'acide aminé. | | |
| Souches | Estimation du nombre de copies de MudAM11 | L-Thr (g/l) |
| EL1016 | 0 | 3,0 |
| EL1012 | 1 | 5,3 |
| EL1013 | 5 (au moins) | 10,2 |

**TABLEAU IX**

| **Activités spécifiques des enzymes de la biosynthèse de la L-thréonine des souches contenant MudAM11** | | |
|---|---|---|
| Le dosage de l'activité aspartokinase-I (AK-I) se fait par la méthode de Truffa-Bachi, P., Cohen, G-N., Methods Enzymology, 1970, 17, 694-702. | | |
| L'activité aspartate semialdehyde déshydrogénase (ASA) est mesurée par la technique de Hegeman, G-D., Cohen, G-N., Margan, R., Methods Enzymology, 1970, 17, 708-713. | | |
| Souches | Activités spécifiques µmole/min/mg de protéines | |
| | AK-I | ASA |
| EL1016 | 4,9 | 0,32 |
| EL1012 | 4,0 | 0,54 |
| EL1013 | 21,1 | 1,14 |

### EXEMPLE 7 : AMPLIFICATION DU GENE aspA D'E.COLI EN VUE DE LA PRODUCTION DE L-ASPARTATE AMMONIA LYASE

### Construction de MudAB9 comprenant le gène aspA codant pour la L-Aspartate ammonia lyase d'E.coli

Le plasmide pGS94 (Guest et al., J. Gen. Microbiol. (1984), 130, 1271-1278) contenant le gène aspA est complètement clivé par les enzymes de restriction ClaI et SalI. Le fragment de 3,4 Kb contient le gène aspA.

Le plasmide pPC3 (résultant de l'inversion du fragment PstI du plasmide pPR3 ; GENE, vol. 42, (1986) pages 185-192) contenant le phage défectif MudPC3 est complètement clivé par les enzymes de restriction ClaI et SalI. Le fragment de 5,3 Kb contient le phage défectif dans lequel on veut cloner le gène aspA.

Les deux plasmides clivés sont mélangés et l'on procède à la ligation à 22°C pendant I heure, au moyen de T4-ADN ligase, d'ATP et de dithiothréitol. Après transformation (exemple 2) de la souche MC1060 avec le mélange de ligation, les transformants sont sélectionnés sur milieu LB contenant 20 µg/ml de Ap et 25 µg/ml de Cm.

27 transformants sont repiqués sur milieu LB contenant 20 µg/ml de Km ; 17 clones sensibles à cet antibiotique sont retenus.

Des dosages de l'activité L-aspartate ammonia-lyase sont effectués sur des extraits acellulaires de cultures de ces clones selon la méthode de Spencer et al., J. Gen. Microbiol. (1976), 97, 73-82. Seul le clone 9 a une activité L-asparate ammonialyase supérieure à l'activité conférée par le gène chromosomique de la souche MC1060.

L'ADN plasmidique de ce clone est extrait par la technique de Birboim et Doly puis clivé par les enzymes de restriction EcoRI et HindIII. En parallèle les ADN des deux plasmides parentaux sont clivés par les mêmes enzymes de restriction. L'analyse des profils de restriction montre que le clone 9 correspond au clonage du fragment contenant le gène aspA dans le phage défectif MudPC3. Le nouveau phage défectif est appelé MudAB9 et le plasmide qui le porte pAB9 (Fig. 17).

### Transposition de MudAB9 sur des ADN chromosomiques

La souche JM109 (Mucts)/pAB9 est obtenue par transformation de la souche d'E.coli JM109 (Mucts) (exemple 2) par le plasmide pAB9.

La souche JM109 (Mucts)/pAB9 est cultivée à 30°C pendant 2 heures. Après avoir subi un choc thermique 45°C pendant 15 minutes, la culture est maintenue à 37°C pendant 2 heures. Quelques gouttes de chloroforme sont alors ajoutées ; après une centrifugation 10 minutes à 5000 tours/min, le lysat de phage II-AB9 est ainsi obtenu.

La souche JM109 (Mucts) est cultivée toute la nuit à 30°C en milieu LB. Du CaCl₂ et du MgSO₄ sont alors ajoutés jusqu'aux concentrations de 1 mM et 2,5 mM respectivement. A 200 µl de cette culture 50µl de lysat de phage II-AB9 sont ajoutés. Après 15 minutes d'adsorption à température ambiante, 2 ml de LB sont ajoutés et les tubes sont agités à 30°C pendant 2 heures. Cette suspension est ensuite étalée sur des boites de milieu LB contenant 25 µg/ml de Cm.

Les boites sont placées à l'étuve à 30°C pendant 24 Heures. 100 colonies sont récoltées et on vérifie leur sensibilité à l'ampicilline. La souche JM109 (Mucts) (MudAB9) est ainsi obtenue. Le lysat de phage III-AB9 est obtenu à partir de cette souche comme décrit précédemment.

A la souche d'E.coli MC4100, cultivée pendant une nuit à 37°C en milieu LB, on ajoute du CaCl₂ et du MgSO₄ jusqu'aux concentrations de 1 mM et 2,5 mM respectivement. La solution de phage III-AB9 est mélangée à 200 µl de cette culture et laissée à température ambiante 15 minutes. Après addition de 2 ml de LB, on continue l'incubation à 30°C pendant 2 heures. La suspension de cellules est étalée sur une boite de milieu LB contenant 25 µg/ml de Cm. La boite est ensuite placée à l'étuve à 30°C pendant 24 heures. Un clone Mu sensible est sélectionné : EL 1010.

### Amplification de MudAB9 sur des ADN chromosomiques

### Détermination du nombre de copies

L'amplification se fait par infection de la souche EL1010 par un lysat de Mucts obtenu comme décrit ci-dessus. Les bactéries sont étalées sur boites de milieu LB contenant 500 µg/ml de Cm.

Les transductants sélectionnés contiennent un nombre stable de copies de MudAB9.

Après incubation à 30°C pendant 48 heures les colonies résistantes sont récoltées et le nombre de copies de phages défectifs MudAB9 est déterminé par la méthode d'hybridation ADN-ADN de Southern (exemple 3).

On étudie particulièrement les souches EL1010 et EL1011.

L'ADN est extrait des souches MC4100, EL1010 et EL1011. Après digestion par l'enzyme de restriction SalI, on soumet l'ADN à une électrophorèse puis on l'absorbe sur une membrane de nylon. L'ADN est ensuite hybridé avec un plasmide marqué par sulfonation contenant le gène aspA.

Sur la figure 18, les canaux A, B et C, correspondent respectivement aux souches MC4100, EL1010 et EL1011.

La lettre "a" correspond au type sauvage du gène aspA.

Dans le canal A, seule la bande "a" correspondant au gène aspA est révélée.

Dans le canal B, deux bandes sont révélées : "a", correspondant au gène aspA et "1" correspondant à une insertion du MudAB9.

Dans le canal C, cinq bandes (au moins) sont révélées : la bande "a", et les bandes "1" à "4" correspondant à 4 insertions (au moins) du MudAB9.

**TABLEAU X**

| **Relation entre le nombre de copies de MudAB9 dans les souches, et leur activité spécifique L-aspartate ammonia-lyase** | | |
|---|---|---|
| Souches | Nombre de copies du MudAB9 | Activité spécifique Δ DO/mn/mg Prot. |
| MC4100 | 0 | 0,33 |
| EL1010 | 1 | 0,60 |
| EL1011 | 4 (au moins) | 2,04 |

### EXEMPLE 8 : PLUSIEURS INSERTIONS EN UNE ETAPE DE LYSOGENISATION

Un lysat de phages est obtenu à partir de la souche JM109 (Mucts) (MudAM9) comme décrit dans l'exemple 2.

A une culture de nuit de la souche d'E.coli : EL 1016 (exemple 6), du CaCl₂ et du MgSO₄ sont ajoutés aux concentrations finales de 1 mM et 2,5 mM respectivement. A 200 µl de cette culture 50 µl de solution de phages III-AM9 sont ajoutés (exemple 2) cette suspension est laissée à température ambiante 15 minutes.

Après addition de 2 ml de LB, l'incubation est de 2 heures à 30°C. La suspension bactérienne est étalée sur boites de milieu LB contenant 250 µg/ml de Km et Nm ; l'incubation est de 24 heures à 30°C. Deux colonies apparues sont testées pour leur lyse à 45°C, et leur sensibilité au phage Mu. Ces deux clones ne lysent pas, sont sensibles à Mu et sont appelés EL1014 et EL1015.

Un test de production de L-Thréonine est réalisé comme décrit dans l'exemple 2. Le nombre de copies de MudAM9 est mesuré par la méthode d'hybridation ADN-ADN de Southern (exemple 3). Les ADN chromosomiques sont digérés par l'enzyme de restriction HindIII. Après électrophorèse, les ADN sont absorbés sur une membrane de nylon puis hybridés avec le fragment (marqué par sulfonation) SalI-BamHI, contenant l'opéron thréonine, du plasmide pAM9. Les résultats concernant la production de L-Thréonine et le nombre de copie de MudAM9 sont consignés dans le tableau XI et la figure 19.

Sur la figure 19, les canaux A, B et C correspondent respectivement aux souches EL1016, EL1014 et EL1015. La lettre "o" correspond au type sauvage de l'opéron thréonine. Chacune des autres bandes correspond à une insertion du MudAM9.

Dans le canal A, seule la bande "o" correspondant à l'opéron thréonine est observée. Dans le canal B, 3 bandes supplémentaires sont observées numérotées de 1 à 3 correspondant à au moins 3 insertions du MudAM9. Dans le canal C, 2 bandes supplémentaires correspondent à 2 insertions du MudAM9 au moins.

**TABLEAU XI**

| Nombre de copies et production de L-Thréonine | | |
|---|---|---|
| Souche | Nombre de copies | Production de L-Thr (g/l) |
| EL1014 | 3 | 6,2 |
| EL1015 | 2 | 5,3 |

### EXEMPLE 9 : STABILITE DE LA LOCALISATION ET DU NOMBRE DE PHAGES DEFECTIFS AU COURS DE LA FERMENTATION

La souche EL1003 (λ⁺, Pro⁻) a été transduite par un phage P1 cultivé sur une souche Pro⁺. Une souche EL1003 (λ⁺, Pro⁺) a été sélectionnée. Le phage λ contenu dans cette souche, a ensuite été éliminé (C. Morel, communication personnelle). La souche ainsi obtenue : EL1003 (λ⁻, Pro⁺) a été appelée EL1017.

La souche EL1017, contenant 10 copies (au moins) de MudAM9, a été testée avant et après fermentation afin de déterminer la stabilité de MudAM9 au cours de cette étape.

Le fermenteur contenant 1,5 litre de milieu de fermentation (voir exemple 2 : composants du milieu) est ensemencé avec la souche EL1017 ; la fermentation a lieu pendant 48 heures à la température appropriée. Des clones sont isolés après fermentation, 4 clones sont particulièrement étudiés : EL1017-a, EL1017-b, EL1017-c et EL1017-d.

La souche mère EL1017 ainsi que les clones EL1017-a, EL1017-b, EL1017-c et EL1017-d sont analysés par la technique d'hybridation ADN-ADN de Southern (exemple 3) permettant de définir la localisation et le nombre de phages défectifs dans les clones testés.

Les ADN chromosomiques des souches EL1016 (exemple 6), EL1017, EL1017-a, EL1017-b, EL1017-c et EL1017-d ont été extraits puis digérés par l'enzyme de restriction HindIII.

Après une électrophorèse, ils sont absorbés sur membrane de nylon et hybridés avec le fragment marqué (par sulfonation) EcoRI-HindIII (contenant les gènes nptII, thrA, thrB et thrC') du plasmide pAM9 (Fig. 1). Le résultat de l'hybridation ADN-ADN est présenté figure 20.

Les canaux A, B, C, D, E et F correspondent respectivement aux souches EL1016, EL1017, EL1017-a, EL1017-b, EL1017-c et EL1017-d.

La lettre "a" correspond au type sauvage de l'opéron thréonine.

Les nombres correspondent aux bandes marquant la spécificité d'hybridation avec MudAM9. Une bande correspond à une insertion de MudAM9.
. Canal A : Seule la bande "a" correspondant à l'opéron thréonine, est révélée.
. Canaux B, C, D, E et F : 10 bandes (au moins) sont révélées, la bande "a" et 9 autres numérotées "1" à "9" correspondant à 9 insertions (au moins) de MudAM9.

Selon ces résultats, les profils d'hybridation de la souche parentale et des clones issus de fermentation sont semblables. Ceci montre la stabilité des phages défectifs insérés dans les chromosomes des bactéries utilisées pour la fermentation.

Les souches suivantes ont été déposées à la Collection Nationale de Cultures de Microorganismes de l'Institut Pasteur, 25 rue du Docteur-Roux à Paris (15ème) le 25 janvier 1988 :
- Escherichia coli K12 POII1681 sous le n° I-727
- Escherichia coli K12 POII1734 sous le n° I-728
- Escherichia coli K12 EL1001 sous le n° I-729
- Escherichia coli K12 EL1002 sous le n° I-730
- Escherichia coli K12 EL1003 sous le n° I-731.

## Revendications

1. procédé pour l'intégration d'un nombre déterminé de copies d'un gène choisi ou d'une séquence d'ADN déterminée dans une séquence d'ADN telle que le chromosome ou l'épisome d'une bactérie, caractérisé en ce que :
a) ledit gène choisi ou la séquence d'ADN choisie est cloné à l'intérieur d'un transposon à l'extérieur des parties essentielles du transposon, ledit transposon étant désarmé,
b) ledit transposon est intégré dans la séquence d'ADN telle que le chromosome ou l'épisome de ladite bactérie,
c) ledit transposon désarmé est complémenté de manière à pouvoir transposer plusieurs fois et la complémentation est ensuite arrêtée après un nombre déterminé de transpositions.

2. Procédé selon la revendication 1, caractérisé en ce que ledit transposon n'est pas capable de transposer.

3. Procédé selon l'une des revendications 1 ou 2 caractérisé en ce que le transposon est dépourvu de transposase.

4. Procédé selon la revendication 3, caractérisé en ce que le transposon n'a pas les gènes codant pour ses transposases.

5. Procédé selon l'une des revendications 1 ou 2,caractérise en ce que l'expression des transposases est réprimée dans ledit transposon.

6. Procédé selon l'une des revendications 1 à S caractérisé en ce que ledit transposon est inséré sur un plasmide et ledit transposon est introduit dans la bactérie par transformation avec ledit plasmide.

7. Procédé selon l'une des revendications 1 à 5, caractérisé en ce que ledit transposon est contenu dans un bactériophage et ledit transposon est introduit dans la bactérie par transduction avec ledit phage.

8. Procédé selon l'une des revendications 1 à 7, caractérisé en ce qu'on intègre un second transposon désarmé dans lequel est cloné un autre gène choisi ou séquence d'ADN déterminée que l'on complémente pour obtenir un nombre de transpositions déterminé.

9. Procédé selon la revendication 8, caractérisé en ce que le transposon est complémenté par transformation de ladite bactérie avec un plasmide exprimant les transposases ou par transduction avec ledit phage.

10. Procédé selon la revendication 9, caractérisé en ce que le transposon est activé après inactivation du répresseur conduisant à l'expression des gènes des transposases.

11. Procédé selon l'une des revendications I à 10, caractérisé en ce que ledit gène choisi est sous le contrôle d'un promoteur qui est aussi à l'intérieur du transposon et qui autorise l'expression dudit gène dans ladite bactérie.

12. Procédé selon l'une des revendications 1 à 11, caractérisé en ce que ledit transposon est choisi parmi le groupe constitué par Tn3, Tn5, Tn7, Tn10, Tn903, TnHoHo, IS 1, IS 10, IS 50, les phages MudI et MudII.

13. Procédé selon l'une des revendications 1 à 12, caractérisé en ce que ladite bactérie appartient à la famille des Enterobacteriacées, des Rhizobiacées ou des Pseudomonas.

14. Procédé selon la revendication 13, caractérisé en ce que ladite bactérie est choisie parmi le groupe constitué par : Escherichia coli, Citrobacter freundii, Erwinia herbicola ou Erwinia chrysanthemi, Salmonella thyphimurium, Shigella sonneii, Enterobacter cloacae, Serratia marcescens, Agrobacterium tumefaciens, Pseudomonas putida.

15. Procédé selon l'une des revendications 1 à 14, caractérisé en ce que ledit transposon code aussi pour un gène marqueur.

16. Procédé selon la revendication 15, caractérisé en ce que ledit gène marqueur est un gène de résistance à un antibiotique s'exprimant dans ladite bactérie.

17. Procédé selon l'une des revendications 1 à 16 caractérisé en ce que le nombre de transpositions est évalué par la détermination de l'expression du gène marqueur.

18. Procédé selon la revendication 17, caractérisé en ce que l'expression du gène marqueur est déterminée par la résistance à un antibiotique quand ledit gène marqueur est un gène de résistance à un antibiotique.

19. Procédé selon la revendication 15, caractérisé en ce que l'expression du gène marqueur est déterminée par la coloration d'un produit quand le gène marqueur (tel que l'opéron lac) est un gène dont l'expression peut être visualisée par un marqueur colorant.

20. Procédé selon l'une des revendications 1 à 19, caractérisé en ce que :
. ledit gène choisi et son promoteur actif dans ladite bactérie hôte sont clonés à l'intérieur d'un phage Mud qui est dépourvu en gênes pour les transposases, dans une partie non essentielle du transposon,
. ledit transposon est intégré à l'intérieur du chromosome d'unc bactérie hôte compatible,
. ledit transposon est complémenté par transformation de l'hôte avec un plasmide codant pour les transposases ou par infection par le phagc Mu,
. après un nombre déterminé de transpositions les souches naturellement curées (sans plasmide ou phage Mu) sont sélectionnées.

21. Procédé selon l'une des revendications 1 à 20 caractérisé en ce que lesdits gènes choisis sont un ou plusieurs gênes de la voie de biosynthèse de la L-thréonine.

22. Procédé selon l'une des revendications 1 à 20, caractérisé en ce que ledit gène choisi code pour un peptide ou une protéine.

23. Souche de bactérie susceptible d'être obtenue par la mise en oeuvre du procédé selon l'une des revendications 1 à 22.

24. Bactérie selon la revendication 23 produisant de la L-thréonine en grande quantité qui comprend dans son chromosome plusieurs copies d'un transposon ayant à l'intérieur tout ou partie des gènes de la voie de biosynthèse de la thréonine.

25. Bactérie selon la revendication 24 caractérisée en ce qu'il s'agit de E. Coli.

26. Procédé pour la préparation des produits de gènes choisis comprenant la culture d'une bactérie selon l'une des revendications 23, 24 et 25 sur un milieu de culture convenable et la récupération desdits produits.

27. Procédé pour la production d'un peptide ou d'une protéine mettant en oeuvre une bactérie selon la revendication 23 sur un milieu de culture convenable et récupération dudit produit.

## Claims

1. Process for integration of a specific number of copies of a chosen gene or of a specific DNA sequence in a DNA sequence such as the chromosome or episome of a bacterium, characterized in that:
a) the said chosen gene or the chosen DNA sequence is cloned inside a transposon outside the essential parts of the transposon, the said transposon being defective,
b) the said transposon is integrated in the DNA sequence such as the chromosome or the episome of the said bacterium,
c) the said defective transposon is complemented so as to be able to transpose several times and the complementation is then stopped after a specific number of transpositions.

2. Process according to Claim 1, characterized in that the said transposon is not capable of transposing.

3. Process according to either of Claims 1 and 2, characterized in that the transposon is deprived of transposase.

4. Process according to Claim 3, characterized in that the transposon does not have the genes coding for its transposases.

5. Process according to either of Claims 1 and 2, characterized in that the expression of the transposases is repressed in the said transposon.

6. Process according to one of Claims 1 to 5, characterized in that the said transposon is inserted on a plasmid and the said transposon is introduced into the bacterium by transformation with the said plasmid.

7. Process according to one of Claims 1 to 5, characterized in that the said transposon is contained in a bacteriophage and the said transposon is introduced into the bacterium by transduction with the said phage.

8. Process according to one of Claims 1 to 7, characterized in that a second defective transposon is integrated in which there is cloned another chosen gene or specific DNA sequence which is complemented in order to produce a specific number of transpositions.

9. Process according to Claim 8, characterized in that the transposon is complemented by transformation of the said bacterium with a plasmid expressing the transposases or by transduction with the said phage.

10. Process according to Claim 9, characterized in that the transposon is activated after inactivation of the repressor leading to the expression of the genes of the transposases.

11. Process according to one of Claims 1 to 10, characterized in that the said chosen gene is under the control of a promoter which is also inside the transposon and which authorizes the expression of the said gene in the said bacterium.

12. Process according to one of Claims 1 to 11, characterized in that the said transposon is chosen from among the group consisting of Tn3, Tn5, Tn7, Tnl0, Tn903, TnHoHo, IS 1, IS 10, IS 50, the MudI and MudII phages.

13. Process according to one of Claims 1 to 12, characterized in that the said bacterium belongs to the family of the Enterobacteriaceae, Rhizobiaceae or Pseudomonas.

14. Process according to Claim 13, characterized in that the said bacterium is chosen from among the group consisting of:
Escherichia coli, Citrobacter freundii, Erwinia herbicola or Erwinia chrysanthemi, Salmonella thyphimurium, Shigella sonneii, Enterobacter cloacae, Serratia marcescens, Agrobacterium tumefaciens, Pseudomonas putida.

15. Process according to one of Claims 1 to 14, characterized in that the said transposon also codes for a marker gene.

16. Process according to Claim 15, characterized in that the said marker gene is an antibiotic-resistant gene expressed in the said bacterium.

17. Process according to one of Claims 1 to 16, characterized in that the number of transpositions is assessed by determination of the expression of the marker gene.

18. Process according to Claim 17, characterized in that the expression of the marker gene is determined by the resistance to an antibiotic when the said marker gene is an antibiotic-resistant gene.

19. Process according to Claim 16, characterized in that the expression of the marker gene is determined by the coloration of a product when the marker gene (such as the lac operon) is a gene the expression of which can be visualized by a colouring marker.

20. Process according to one of Claims 1 to 19, characterized in that:
. the said chosen gene and its active promoter in the said host bacterium are cloned inside a Mud phage which is deprived of genes for the transposases, in a non-essential part of the transposon,
. the said transposon is integrated inside the chromosome of a compatible host bacterium,
. the said transposon is complemented by transformation of the host with a plasmid coding for the transposases or by infection by the Mu phage,
. after a specific number of transpositions, the naturally cured strains (without plasmid or Mu phage) are selected.

21. Process according to one of Claims 1 to 20, characterized in that the said chosen genes are one or several genes from the biosynthesis pathway of L-threonine.

22. Process according to one of Claims 1 to 20, characterized in that the said chosen gene codes for a peptide or a protein.

23. Bacterium strain which can be obtained by implementation of the process according to one of Claims 1 to 22.

24. Bacterium according to Claim 23, producing L-threonine in large quantities, which comprises in its chromosome several copies of a transposon having inside it all or some of the genes from the biosynthesis pathway of threonine.

25. Bacterium according to Claim 24, characterized in that it is E. coli.

26. Process for the preparation of the products of chosen genes comprising the culturing of a bacterium according to one of Claims 23, 24 and 25 in a suitable culture medium and the recovery of the said products.

27. Process for the production of a peptide or a proteinby culturing a bacterium according to Claim 23 in a suitable culture medium and recovering the said product.

## Patentansprüche

1. Verfahren zur Integration einer vorgegebenen Anzahl von Kopien eines ausgewählten Gens oder einer vorgegebenen DNA-Sequenz in eine DNA-Sequenz wie das Chromosom oder das Episom eines Bakteriums, dadurch gekennzeichnet, daß:
a) das ausgewählte Gen oder die ausgewählte DNA-Sequenz im Innern eines Transposons außerhalb der wesentlichen Teile des Transposons kloniert wird, wobei das Transposon entspannt ist,
b) das genannte Transposon in die DNA-Sequenz wie das Chromosom oder das Episom des genannten Bakteriums integriert wird und
c) das genannte entspannte Transposon so komplementiert wird, daß es mehrmals transponiert werden kann, und die Komplementierung anschließend nach einer vorgegebenen Anzahl von Transpositionen beendet wird.

2. Verfahren nach Anspruch 1, dadurch gekennzeichnet, daß das genannte Transposon zur Transposition nicht in der Lage ist.

3. Verfahren nach einem der Ansprüche 1 oder 2, dadurch gekennzeichnet, daß das Transposon frei von Transposase ist.

4. Verfahren nach Anspruch 3, dadurch gekennzeichnet, daß das Transposon keine für seine Transposasen codierenden Gene aufweist.

5. Verfahren nach einem der Ansprüche 1 oder 2, dadurch gekennzeichnet, daß die Expression der Transposasen in dem genannten Transposon reprimiert wird.

6. Verfahren nach einem der Ansprüche 1 bis 5, dadurch gekennzeichnet, daß das genannte Transposon auf einem Plasmid inseriert wird und das genannte Transposon durch Transformation mit dem genannten Plasmid in das Bakterium eingeführt wird.

7. Verfahren nach einem der Ansprüche 1 bis 5, dadurch gekennzeichnet, daß das genannte Transposon in einem Bakteriophagen enthalten ist und daß das genannte Transposon durch Transduktion mit dem genannten Phagen in das Bakterium eingeführt wird.

8. Verfahren nach einem der Ansprüche 1 bis 7, dadurch gekennzeichnet, daß man ein zweites entspanntes Transposon integriert, in dem ein anderes ausgewähltes Gen oder eine vorgegebene DNA-Sequenz kloniert wird, die man komplementiert, um eine vorgegebene Anzahl von Transpositionen zu erhalten.

9. Verfahren nach Anspruch 8, dadurch gekennzeichnet, daß das Transposon durch Transformation des genannten Bakteriums mit einem Plasmid, das die Transposasen exprimiert, oder durch Transduktion mit dem genannten Phagen komplementiert wird.

10. Verfahren nach Anspruch 9, dadurch gekennzeichnet, daß das Transposon nach der Inaktivierung des Repressors aktiviert wird, was zur Expression der Gene der Transposasen führt.

11. Verfahren nach einem der Ansprüche 1 bis 10, dadurch gekennzeichnet, daß das genannte ausgewählte Gen unter der Kontrolle eines Promotors steht, der sich ebenfalls im Innern des Transposons befindet und der die Expression des genannten Gens in dem genannten Bakterium erlaubt.

12. Verfahren nach einem der Ansprüche 1 bis 11, dadurch gekennzeichnet, daß das genannte Transposon ausgewählt wird aus der Gruppe, die besteht aus Tn3, Tn5, Tn7, Tn10, Tn903, TnHoHo, IS 1, IS 10, IS 50, den Phagen Mudl und Mudll.

13. Verfahren nach einem der Ansprüche 1 bis 12, dadurch gekennzeichnet, daß das genannte Bakterium zur Familie der Enterobacteriaceae, der Rhizobiaceae oder der Pseudomonaden gehört.

14. Verfahren nach Anspruch 13, dadurch gekennzeichnet, daß das genannte Bakterium ausgewählt wird aus der Gruppe, die besteht aus Escherichia coli, Citrobacter freundii, Erwinia herbicola oder Erwinia chrysanthemi, Salmonella thyphimurium, Shigella sonneii, Enterobacter cloacae, Serratia marcescens, Agrobacterium tumefaciens und Pseudomonas putida.

15. Verfahren nach einem der Ansprüche 1 bis 14, dadurch gekennzeichnet, daß das genannte Transposon auch für ein Marker-Gen codiert.

16. Verfahren nach Anspruch 15, dadurch gekennzeichnet, daß das genannte Marker-Gen ein Antibiotikum-Resistenz-Gen ist, das sich in dem genannten Bakterium exprimiert.

17. Verfahren nach einem der Ansprüche 1 bis 16, dadurch gekennzeichnet, daß die Anzahl der Transpositionen durch die Bestimmung der Expression des Marker-Gens bewertet wird.

18. Verfahren nach Anspruch 17, dadurch gekennzeichnet, daß die Expression des Marker-Gens durch die Resistenz gegenüber einem Antibiotikum bestimmt wird, wenn das genannte Marker-Gen ein Antibiotikum-Resistenz-Gen ist.

19. Verfahren nach Anspruch 16, dadurch gekennzeichnet, daß die Expression des Marker-Gens durch die Färbung eines Produkts bestimmt wird, wenn das Marker-Gen (z.B. das Operon lac) ein Gen ist, dessen Expression durch einen färbenden Marker sichtbar gemacht werden kann.

20. Verfahren nach einem der Ansprüche 1 bis 19, dadurch gekennzeichnet, daß
- das ausgewählte Gen und sein aktiver Promotor in dem genannten Wirts-Bakterium im Innern eines Phagen Mud, der frei von Genen für die Transposasen ist, in einem nicht-wesentlichen Teil des Transposons kloniert werden,
- das genannte Transposon in das Innere des Chromosoms eines kompatiblen Wirts-Bakterium integriert wird,
- das genannte Transposon durch Transformation des Wirts mit einem Plasmid, das für die Transposasen codiert, oder durch Infektion durch den Phagen Mu komplementiert wird, und
- nach einer bestimmten Anzahl von Transpositionen die in natürlicher Weise kurierten Stämme (ohne Plasmid oder den Phagen Mu) selektioniert werden.

21. Verfahren nach einem der Ansprüche 1 bis 20, dadurch gekennzeichnet, daß es sich bei den ausgewählten Genen um ein oder mehrere Gene des Weges zur Biosynthese von L-Threonin handelt.

22. Verfahren nach einem der Ansprüche 1 bis 20, dadurch gekennzeichnet, daß das ausgewählte Gen für ein Peptid oder ein Protein codiert.

23. Bakterienstamm, der durch Anwendung des Verfahrens nach einem der Ansprüche 1 bis 22 erhalten werden kann.

24. Bakterium nach Anspruch 23, welches das L-Threonin in großer Menge bildet, das in seinem Chromosom mehrere Kopien eines Transposons enthält, das im Innern vollständige oder Teile der Gene des Weges zur Biosynthese von Threonin aufweist.

25. Bakterium nach Anspruch 24, dadurch gekennzeichnet, daß es sich dabei um E. coli handelt.

26. Verfahren zur Herstellung von ausgewählten Gen-Produkten, das umfaßt die Kultivierung eines Bakteriums nach einem der Ansprüche 23, 24 und 25 auf einem geeigneten Kulturmedium und die Gewinnung (Abtrennung) der genannten Produkte.

27. Verfahren zur Herstellung eines Peptids oder eines Proteins, bei dem man ein Bakterium nach Anspruch 23 auf einem geeigneten Kulturmedium kultiviert und das genannte Produkt gewinnt (abtrennt).
